(19) Europäisches Patentamt
European Patent Office
Office européen des brevets

(11) **EP 0 967 280 B1**

(12) **FASCICULE DE BREVET EUROPEEN**

(45) Date de publication et mention
de la délivrance du brevet:
**12.08.2009 Bulletin 2009/33**

(51) Int Cl.:
*C12N 15/31* (2006.01)   *C12N 15/60* (2006.01)
*C12N 9/88* (2006.01)   *C07K 14/395* (2006.01)
*C12N 1/18* (2006.01)   *C12Q 1/02* (2006.01)
*C12R 1/865* (2006.01)   *A21D 8/04* (2006.01)
*A21D 6/00* (2006.01)

(21) Numéro de dépôt: **99401444.7**

(22) Date de dépôt: **11.06.1999**

(54) **Nouvelles souches "fil" résistantes au stress en condition de fermentation et/ou de croissance**

Hefe mit fil Phenotyp, stress-resistent unter Wachstum- und/oder Gährungsbedingungen

Yeast strains with fil phenotype, stress resistant under conditions of growth and/or fermentation

(84) Etats contractants désignés:
**AT BE CH DE DK ES FI FR GB GR IE IT LI LU NL PT SE**
Etats d'extension désignés:
**LT LV RO SI**

(30) Priorité: **12.06.1998 FR 9807463**

(43) Date de publication de la demande:
**29.12.1999 Bulletin 1999/52**

(73) Titulaire: **LESAFFRE et Cie**
**F-75001 Paris (FR)**

(72) Inventeurs:
• **Thevelein, Johan**
**3001 Heverlee (BE)**
• **Gorwa, Marie-Françoise**
**59800 Lille (FR)**
• **Van Dijck, Patrick**
**3271 Zichem (BE)**
• **Dumortier, Françoise**
**3001 Heverlee (BE)**
• **Teunissen, Aloys**
**3001 Heverlee (BE)**
• **Lemaire, Katleen**
**3001 Heverlee (BE)**
• **Colavizza, Didier**
**59163 Conde Sur Escaut (FR)**
• **Versele, Matthias**
**3550 Zolder (BE)**
• **Loiez, Annie**
**59800 Lille (FR)**

(74) Mandataire: **Koch, Gustave**
**Cabinet Plasseraud**
**52 rue de la Victoire**
**75440 Paris Cedex 09 (FR)**

(56) Documents cités:
**EP-A- 0 196 233**     **EP-A- 0 487 878**
**JP-A- 08 051 917**

• **RO 111471 B (INST. CERC. CHIM. BUCURESTI) 31 octobre 1996 XP002095522**
• **PARK J.I. ET AL.: "The freeze-thaw stress response of the yeast Saccharomyces cerevisiae is growth phase specific and is controlled by nutritional state via the RAS-cyclic AMP signal transduction pathway" APPL. ENVIRONM. MICROBIOL., vol. 63, no. 10, octobre 1997 (1997-10), pages 3818-3824, XP002095496**
• **IIDA H.: "Multistress resistance of Saccharomyces cerevisiae is generated by insertion of retrotransposon Ty into the 5' coding region of the adenylate cyclase gene" MOL. CELL. BIOL., vol. 8, no. 12, décembre 1988 (1988-12), pages 5555-5560, XP002095497**
• **YUN C.W. ET AL.: "G-protein-coupled receptor from yeast S. cerevisiae" BIOCHEM. BIOPHYS. RES. COMM., vol. 240, 1997, pages 287-292, XP002095499**

**Description**

La présente invention a pour objet des levures présentant le phénotype fil, c'est-à-dire ayant la propriété inattendue de conserver une résistance importante au stress en phase de fermentation et/ou de croissance, alors qu'elles ont gardé un métabolisme respiratoire et un métabolisme fermentaire normaux sur sucre fermentescible tel le glucose. Un autre objet de l'invention est le procédé d'obtention de telles souches. Encore un objet de l'invention est l'utilisation de telles souches pour l'obtention de levures de boulangerie plus résistantes au séchage, plus adaptées à la préparation de pâtes congelées et/ou pour d'autres applications où une forte résistance au stress durant la phase de fermentation est recherchée.

Les levures du genre Saccharomyces sont utilisées comme agent de fermentation en boulangerie, en brasserie, vinification, distillerie et dans d'autres domaines. Leur utilisation industrielle repose sur leur capacité à produire du dioxyde de carbone à partir des sucres comme le glucose, le fructose, le saccharose ou le maltose, présents ou ajoutés dans la pâte ou dans le moût. Le pouvoir fermentatif est un critère important de qualité de la levure.

La sélection des souches, les conditions de production des levures vivantes ont été optimisées au fil des années de manière à obtenir des levures ayant une bonne capacité fermentaire et une bonne résistance au stress dans certaines conditions. Malheureusement, si les cellules de levure récoltées dans des conditions équivalentes à celles d'une phase stationnaire montrent un niveau de résistance élevé aux différents types de stress tels que la chaleur, la congélation et le séchage, cette résistance au stress est perdue quand on initie une phase de fermentation par ajout de sucre fermentescible. Les cellules perdent alors rapidement leur propriété de résistance au stress, ce qui entraîne une réduction de leur potentiel fermentatif dans des conditions de stress, ce qui est un inconvénient majeur dans la plupart des applications industrielles des levures.

La présente invention est relative à de nouvelles souches eucaryotes et notamment à de nouvelles souches de levure pouvant être obtenues par mutation ou transformation par ADN recombinant, appelées souches " fil". L'emploi des lettres minuscules signifie qu'elles n'ont pas le phénotype FIL qui est le phénotype normal signifiant "Fermentation-Induced Loss of stress resistance", c'est-à-dire perte de résistance au stress induite par la fermentation. Les nouvelles souches fil ont un phénotype "deficient in Fermentation-Induced Loss of stress resistance" c'est-à-dire qu'elles ont une déficience dans la perte de résistance au stress induite par la fermentation, sans perte ou altération gênante de leur potentiel de fermentation et/ou de croissance. En d'autres termes les nouvelles souches conservent en phase de métabolisme actif un haut niveau de résistance au stress, comparable à celui des cellules qui ne sont pas en fermentation ou en croissance, c'est-à-dire qui sont en phase stationnaire. Cette propriété nouvelle des nouvelles souches est d'autant plus inattendue que le maintien d'une forte résistance au stress est obtenu sans perte simultanée significative de leurs capacités de croissance et de fermentation.

Tous les organismes unicellulaires eucaryotes (levures, moisissures, ...) sont confrontés à des conditions de stress alors que leur utilisation industrielle exige qu'ils soient en phase métabolique active ou qu'ils puissent entrer très rapidement dans un métabolisme très actif. Ces deux exigences, résistance au stress et métabolisme actif, ont toujours été considérées comme contradictoires et contraires à l'équilibre biologique naturel. Par exemple, Attfield, dans sa revue sur la résistance au stress des levures du genre Saccharomyces publiée en décembre 1997 dans Nature Biotechnology, 15, pp.1351-1357, écrit que la conciliation de ces deux exigences est contraire au " biological design " c'est-à-dire à l'équilibre naturel fondamental ou encore à la conception biologique originelle des souches de levure. En conséquence, l'obtention de nouvelles souches eucaryotes et notamment de levures présentant les propriétés inattendues du phénotype fil représente un progrès considérable. Si on prend l'exemple des levures de panification, il est bien connu que plus une biomasse cellulaire aura été récoltée dans des conditions proches d'une phase exponentielle de croissance, plus son potentiel fermentatif sera élevé, mais moins elle résistera au stress d'un séchage ou d'une congélation. Il est en outre bien connu que même si on ensemence des pâtons panaires destinés à être congelés avec une biomasse récoltée dans des conditions proches de la phase stationnaire et en conséquence résistante au stress, la reprise de fermentation dans le pâton dès le pétrissage et pendant tout le temps avant la congélation à coeur du pâton induira une perte de cette résistance au stress. Les nouvelles souches ayant le phénotype fil permettent d'apporter un progrès à la résolution de ces difficultés inhérentes à l'équilibre fondamental biologique des cellules eucaryotes.

De nombreux travaux ont été réalisés ces dernières années pour comprendre les mécanismes d'action des facteurs de stress sur la cellule et la nature de la réponse cellulaire, notamment chez *Saccharomyces cerevisiae,* quand la levure est soumise à différents stress chimiques ou physiques. En général, la résistance apparaît comme un phénomène complexe impliquant plusieurs facteurs jouant des rôles distincts. Ces facteurs, leurs mécanismes d'action et leur importance relative sont encore mal compris.

Il est bien connu que les cellules de la levure *Saccharomyces cerevisiae* deviennent résistantes au stress durant la phase stationnaire ou quand elles sont cultivées à un niveau de croissance faible sur une source de carbone non fermentescible ou avec un apport limité en sucre fermentescible et/ou en azote. Cependant, cette forte résistance au stress disparaît quand un sucre fermentescible tel que le glucose ou le maltose est apporté aux cellules qui entrent alors en fermentation rapide ou en croissance (Attfield, 1997, Nature Biotechnology, 15, pp.1351-1357 ; de Winde et al., 1997, Yeast Stress Responses, Ed. Springer, pp.7-52 ; Werner-Washburne et al., 1993, Microbiol.Rev., 57, pp. 383-401).

La voie métabolique Ras-AMPc (protéines Ras - Adénosine MonoPhosphate cyclique) est connue pour son influence dramatique sur la résistance à différents types de stress dans les cellules de levure. Ceci a été montré en ce qui concerne la résistance à la chaleur (Iida, 1988, Mol. Cell. Biol., 8, pp.5555-5560; Matsumoto et al., 1985, Yeast, 1, pp.15-24 ; Shin et al., 1987, Mol. Cell. Biol., 7, pp.244-250), en ce qui concerne la résistance à des congélations et décongélations successives (Park et al., 1997, Appl. Envir. Microbiol., 63, pp.3818-3824), et en ce qui concerne la résistance au sel (Hitara et al., 1995, Mol. Gen. Genet., 249, pp.257-264).

C'est une voie élaborée et complexe qui contrôle le niveau d'AMPc et en conséquence l'activité de la protéine kinase A des cellules de levure (Broach et Deschenes, 1990, Adv. Cancer Res., 54, pp.79-139 ; Thevelein, 1991, Mol. Microbiol., 5, pp.1301-1307 ; Thevelein, 1992, Antonie Leeuwenhoek, J. Microbiology, 62, pp.109-130). Dans la levure, l'AMPc est synthétisée par une enzyme, l'adénylate cyclase, qui est codée par le gène *CYR1/CDC35* (Kataoka et al., 1985, Cell, 43, pp.493-505). Le niveau de l'AMPc est notamment régulé par deux phosphodiestérases, codées par les gènes *PDE1* et *PDE2* et qui l'hydrolysent (Nikawa et al., 1997, Mol. Cell. Biol., 7, pp.3629-3636; Sass et al., 1986, Proc. Natl. Acad. Sci. USA, 83, pp.9303-9307). Par ailleurs, l'activité de l'adénylate cyclase est fortement dépendante de l'activité des protéines Ras (Toda et al., 1985, Cell, 40, pp.27-36).

Les protéines Ras sont des protéines G. Elles sont actives quand elles sont liées à une GTP (Guanosine TriPhosphate), et inactives dans un état lié à une GDP (Guanosine DiPhosphate). L'échange de GDP en GTP sur les protéines Ras est stimulé par les protéines Cdc 25 et Sdc 25 d'échange des nucléotides guanine (Boy-Marcotte et al., 1996, Mol. Biol. Cell, 7, pp.529-539 ; Camonis et al., 1986, EMBO J., 5, pp.375-380). Les protéines Ras possèdent une activité GTPase intrinsèque, qui est stimulée par les protéines Ira1 et Ira2 et qui est responsable de la régulation aval de leur activité. L'AMPc active la protéine kinase A AMPc dépendante (protéine kinase A dépendante de l'AMPc appelée ci-après PKA) qui est composée de sous-unités catalytiques codées par les gènes *TPK1, TPK2* et *TPK3* et d'une sous-unité de régulation codée par le gène *BCY1* (Toda et al., 1987, Mol. Cell. Biol., 7, pp.1371-1327; Toda et al., 1987, Cell, 50, pp.277-287). La liaison de l'AMPc à la sous-unité inhibitrice Bcyl entraîne la dissociation de sa liaison avec lesdites sous-unités catalytiques, ce qui entraîne l'activation de ces sous-unités catalytiques. Celles-ci sous forme activée phosphorylent un certain nombre de protéines cibles dont quelques unes ont été identifiées, telle la tréhalase. L'activité de la PKA (protéine kinase A activée par l'AMPc) est essentielle pour la croissance des cellules de levure. Lorsque l'activité de la PKA est d'une manière ou d'une autre fortement réduite, par exemple par une forte diminution de l'AMPc, les cellules arrêtent leur phase de croissance et entrent de manière permanente en phase stationnaire.

En d'autres termes, la PKA, quand elle est activée, entraîne la croissance des levures et est un médiateur de différents processus de régulation métabolique entraînant notamment une baisse rapide de la teneur en tréhalose et une baisse rapide de la teneur en protéines de choc thermique, c'est-à-dire une disparition de facteurs favorables à la résistance au stress. Au contraire, lorsque l'activité de la PKA est fortement réduite, les cellules entrent en phase stationnaire et acquièrent une forte résistance au stress. Comme indiqué ci-dessus, cela a été montré pour différents types de stress.

C'est l'étude de ces mécanismes de la voie métabolique complexe Ras-AMPc qui a conduit Attfield à établir que l'obtention d'un phénotype correspondant au maintien d'une forte résistance au stress pour des cellules en métabolisme actif après inoculation sur un milieu contenant des sucres fermentescibles allait contre le " biological design " des souches, c'est-à-dire contre l'équilibre naturel.

Des mutants dans la voie Ras-AMPc-protéine kinase A ont été identifiés, ayant constitutivement une résistance au stress élevée durant la croissance. Ceci a été montré en ce qui concerne la résistance à la chaleur (Cameron et al., 1988, Cell, 53, pp.555-566 ; Hottiger et al., 1989, FEBS Lett., 255, pp.431-434; Shin et al., 1987, Mol. Cell. Biol., 7, pp. 244-250) et à la congélation/décongélation (Park et al., 1997, Appl. Envir. Microbiol., 63, pp.3818-3824). Cependant de tels mutants ont une phase de latence beaucoup plus longue lors du démarrage de la fermentation et/ou un taux de croissance réduit (Ma et al., 1997, Microbiol., 143, pp.3451-3459 ; Iida, 1988, Mol. Cell. Biol., 8, page 5559), ce qui exclut leur utilisation industrielle notamment en levures de boulangerie où un départ rapide en fermentation est essentiel.

Certains de ces mutants voient d'autres propriétés importantes pour leur utilisation industrielle affectées négativement. Par exemple, les mutants *ras2* sont incapables d'utiliser pour leur croissance une source de carbone non fermentescible, l'éthanol par exemple (Tatchell et al., 1985, Proc. Natl. Acad. Sci., 82, pp.3785-3789, J.F. Canon et al, Genetics 113, pp. 247-264, June 1986). Comme l'assimilation de l'éthanol est nécessaire pour la culture des levures de boulangerie, il est exclu d'utiliser une levure ayant une disruption du gène *RAS2* comme levure de boulangerie.

En d'autres termes, il semble que ces mutants gardent constitutivement une forte résistance au stress car ils sont incapables de rentrer en phase métabolique vraiment active.

A l'opposé, les mutants dérégulés ayant un niveau élevé d'AMPc ou une activité PKA non limitée montrent un niveau très faible à la fois des protéines de choc thermique et du tréhalose, quelles que soient les conditions de culture et donc également en phase stationnaire.

L'étude de ces mutants qui n'ont aucun intérêt industriel confirme bien les conclusions de la revue générale d'Attfield déjà citée sur la résistance au stress, selon laquelle par génétique classique il paraît improbable d'obtenir des souches industrielles utiles et qu'il faut alors se tourner vers les technologies d'ADN recombinant. Cependant les données théoriques permettant une telle approche sont insuffisantes pour obtenir le résultat nouveau recherché. Ceci est notamment illustré par l'ensemble des travaux sur la délétion du ou des gènes codant pour une tréhalase.

Il est bien connu que chez la levure de boulangerie une forte résistance au stress, par exemple à la chaleur, à la congélation ou aux hautes pressions, est corrélée à une teneur élevée en tréhalose (Attfield, 1997, Nature Biotechnology, 15, pp.1351-1357 ; De Virgilio et al, 1994, Eur. J. Biochem., 219, pp.179-186 ; Iwakashi et al., 1997, Lett. Appl. Microbiol., 25, pp.43-47; Wiemken, 1990, Antonie Leeuwenhoek, J. Microbiology, 58, pp.209-217). Le tréhalose est un dioside présent à de fortes concentrations dans de nombreuses formes vivantes dans la nature (Van Laere, 1989, FEMS Microbiol. Rev., 63, pp.201-210; Wiemkem 1990, Antonie Leeuwenhoek, J. Microbiology, 58, pp.209-217). Il possède des propriétés remarquables et apparemment spécifiques de protection contre les traitements agressifs pour toute une série de structures biologiques (Crowe et al., 1992, Anhydrobiosos Annu. Rev. Physiol., 54, pp.579-599). Le tréhalose est accumulé rapidement durant la phase précédant la mort des cellules de levure.

L'initiation de la fermentation par addition d'une source de carbone fermentescible est associée à une mobilisation rapide du tréhalose (van der Plaat, 1974, Biochem. Biophys. Res. Commun., 56, pp.580-587), c'est-à-dire à son utilisation métabolique et à sa disparition rapide. Ainsi, une approche logique pour maintenir la résistance au stress durant le démarrage de la fermentation a été de cloner et de déléter le gène *NTH1,* codant pour la tréhalase neutre (Kopp et al., 1993, J. Biol. Chem., 268, pp.4766-4774) qui est l'enzyme responsable de la mobilisation du tréhalose, de manière à conserver le tréhalose des cellules. Il a été revendiqué que la résistance au stress de la levure pouvait être améliorée par la délétion de ce gène (demandes de brevet ou brevets EP 0451896 ; Hino et al., EP 0838520). Cependant, en empêchant la mobilisation du tréhalose par la délétion du gène *NTH1,* on n'évite pas la perte rapide de résistance au stress durant l'initiation de la fermentation (Van Dijck et al., 1995, Appl. Environ. Microbiol., 61, pp.109-115). Ceci est également vrai concernant la non expression du gène *ATH1* revendiquée dans la demande WO 97/01626. La simple délétion d'un ou de l'ensemble des gènes codants pour une tréhalase n'est pas à elle seule susceptible de résoudre le problème objet de la présente invention.

Ceci est probablement dû notamment à la mise en oeuvre d'autres facteurs de résistance tels que les protéines de choc thermique, qui disparaissent aussi rapidement au début de la fermentation (Crauwels et al., 1997, Microbiol. 143, pp.2627-2637 ; de Winde et al., 1997, Yeast Stress Responses, Ed. Springer, pp.7-52 ; Praekelt et Maecock, 1990, Mol. Gen. Genet., 223, pp.97-106 ; Werner-Washburne et al., 1989, J. Bacteriol., 171, pp.2680-2688).

Ainsi, la modification du métabolisme du tréhalose par les méthodes de génie génétique n'a pas permis d'améliorer la résistance au stress des levures et n'a pas donné de résultats pratiques pour le développement de souches industrielles résistantes au stress durant la phase de fermentation (Attfield, 1997, Nature Biotechnology, 15, pp. 1351-1357).

Une autre approche utilisant les techniques d'ADN recombinant ou génie génétique a été d'essayer d'augmenter la résistance au stress des levures par production de protéines antigel présentes dans le sang de certains poissons vivant dans des eaux très froides. McKown et al., (Cryobiology, 1991, 28, pp.474-482) ont exprimé le gène codant pour une protéine antigel dans *Saccharomyces cerevisiae* afin de lui faire produire de manière intracellulaire une protéine chimère antigel. Cependant cette approche n'a pas donné de résultats satisfaisants, car le taux de survie de la levure après congélation est encore très faible.

On peut également citer de nombreux travaux japonais (demandes de brevet ou brevets EP 196233, US 4 547 374, EP 0 388 262) datant de la décennie 1980-1989 qui ont consisté à sélectionner des souches non conventionnelles pour la fermentation panaire mais ayant des propriétés intéressantes de résistance à la congélation, et à les croiser avec des levures de panification. Cette approche a eu des résultats limités ne résolvant pas le problème objet de la présente invention et on ne retrouve aucune de ces souches en 1998 sur le marché européen ou américain.

JP 0805 1917 décrit une méthode de sélection de levures possédant une activité fermentative importante à 30°C dans une pâte contenant des analogues du sucre, et une activité fermentative basse à 5°C, ce qui ne correspond pas à des conditions de choc thermique induisant un stress élevé.

Ainsi, il n'a pas été possible jusqu'à présent d'obtenir des levures ayant la propriété de conserver une résistance au stress élevée simultanément à une bonne croissance et une bonne activité fermentative.

En conclusion, on peut dire, en accord notamment avec les conclusions sur la revue générale sur le stress des levures publiée dans Nature Biotechnology en 1997, que :

- le problème majeur est que la réponse naturelle de cellules, quand elles sont mises en présence d'un substrat fermentescible, est de passer en phase métabolique active et donc d'abaisser très rapidement leurs facteurs de résistance au stress, alors que les conditions industrielles nécessitent métabolisme actif et forte résistance au stress ;
- ce problème était non résolu ;
- les travaux de génétique classique n'ont apporté qu'une amélioration limitée de la résistance des cellules en conditions d'utilisation industrielle ;

- une solution de ce problème non résolu semblait seulement pouvoir être espérée à travers les technologies du génie génétique.

Malheureusement, en dépit de la connaissance complète du génome de la levure qui a été entièrement séquencé et des connaissances qui s'accumulent sur les fonctions des gènes mais qui sont encore très incomplètes, on ne connaît que très partiellement comment les propriétés industriellement décisives sont gouvernées génétiquement et physiologiquement. Les connaissances actuelles sont donc insuffisantes pour permettre des manipulations génétiques adéquates pour arriver à une solution du problème majeur ci-dessus défini. Ceci est d'autant plus vrai que les voies métaboliques concernées comme la voie Ras-AMPc sont très complexes comme illustré ci-dessus, et sont probablement nombreuses. La voie Ras-AMPc-PKA n'est pas la seule voie La présente invention de manière étonnante est arrivée à surmonter ces problèmes d'une façon à la fois simple et efficace. Elle montre qu'il est possible contrairement à ce qui était généralement admis, d'obtenir des souches ayant un phénotype dont il était cru qu'il ne pouvait pas exister. Ce phénotype contraire au " biological design " (c'est-à-dire à l'équilibre naturel) a été appelé phénotype fil.

## BREVE DESCRIPTION DE L'INVENTION

La présente invention a pour objet un procédé d'obtention de souches de levure, et encore de préférence des souches de *Saccharomyces cerevisiae* conservant une résistance au stress en présence de sucre fermentescible tel que le glucose, caractérisé par le fait qu'il comprend les étapes suivantes :

on soumet les cellules d'une souche de départ à un traitement mutagène classique,
on cultive les cellules ayant subi ledit traitement mutagène jusqu'à obtention d'une phase stationnaire,
on incube lesdites cellules en phase stationnaire en présence d'au moins un sucre fermentescible choisi dans le groupe comprenant ie glucose, le maltose et le saccharose, ce sucre étant présent en une quantité déterminée de manière à ce que les cellules passent en état de métabolisme actif (fermentation et/ou croissance) de ce sucre,
on soumet lesdites cellules en état de métabolisme actif à un ou plusieurs stress conduisant à un taux de mortalité d'au moins 99% par rapport à la population de départ,
on isole les cellules survivantes et
on sélectionne celles de ces cellules survivantes qui répondent aux critères suivants qui caractérisent le phénotype fil, à savoir :

- une croissance, évaluée par une production ou un rendement de production de biomasse sur sucre dans un temps donné ou par un taux de croissance, en conditions identiques de culture, au moins égale à 80% de la souche de départ, et de préférence au moins égale à 90% de la souche de départ,
- un dégagement de $CO_2$, ou une production de métabolite, en conditions identiques, au moins égal à 80%, et de préférence au moins égal à 90% de la souche de départ,
- une résistance au stress, correspondant à un taux de survie au moins 2 fois supérieur, de préférence au moins 3 fois supérieur, plus préférentiellement au moins 5 fois supérieur, et encore plus préférentiellement au moins 10 fois supérieur au taux de survie de la souche de départ, dans des conditions identiques de phase correspondant à une croissance ou un métabolisme actif suivi d'un choc thermique d'au moins 20 minutes à 52˚C, et/ou au moins 1,5 fois supérieur, de préférence au moins 2 fois supérieur, plus préférentiellement au moins 3 fois supérieur, et encore plus préférentiellement au moins 5 fois supérieur au taux de survie de la souche de départ, dans des conditions identiques de phase de croissance ou de fermentation suivies d'une congélation d'une durée d'au moins 24 heures à -20˚C ou à une température inférieure,
- maintien de l'ensemble de ces propriétés après cultures répétées sur milieu non sélectif, tel que le milieu YPD, de manière à vérifier que le phénotype fil obtenu par mutation est parfaitement stable et permanent.

De préférence, on recherchera dans le cadre du phénotype fil, l'absence de toutes propriétés gênantes pour leur utilisation. Ces propriétés gênantes peuvent être par exemple la formation de métabolites secondaires inopportuns, ou la perte de la capacité d'assimiler ou de fermenter certains composés.

Suivant une particularité de l'invention, les souches fil sélectionnées ont de préférence la propriété de conserver, en phase de croissance et/ou fermentation sur sucres fermentescibles, au moins 50%, de préférence au moins 60%, plus préférentiellement au moins 70%, et encore plus préférentiellement au moins 80% de leur taux de survie par rapport au taux de survie en phase stationnaire mesuré dans de mêmes conditions après un choc à la chaleur ou à la congélation.

Dans une forme particulière de réalisation du protocole d'obtention des nouvelles souches fil, les cellules obtenues après un traitement de mutagenèse et en phase stationnaire sont introduites dans des pâtons de 0,5g composés d'eau (environ 42,5%), de farine (environ 56,5%), de NaCl (environ 1%), à raison de $4.10^8$ cellules par g de pâton. Lesdits pâtons subissent une première fermentation de 30 minutes à 30˚C, puis sont soumis à au moins 100 cycles de congélation/décongélation. De préférence, le procédé d'obtention de souches objet de l'invention est mis en oeuvre directement sur des souches industrielles. Les tests de sélection employés correspondent aux stress rencontrés par ladite souche dans le ou les procédés de fabrication dans lesquels elle est utilisée et aux caractéristiques de performance de cette souche. La souche présentant le phénotype fil qui est sélectionnée est une souche ayant à la fois les caractéristiques justifiant son emploi en fabrication industrielle ou artisanale et une meilleure résistance aux stress rencontrés. Une souche industrielle est une souche effectivement employée dans une production industrielle optimisée et compétitive. Si la souche de départ est une souche de levure de panification, cette souche est alors une souche effectivement utilisée par un producteur spécialisé de levures pour la mise sur le marché de levures de boulangerie, ou une souche ayant des propriétés équivalentes.

Une souche de laboratoire est une souche modèle permettant de bien comprendre les phénomènes étudiés, mais qui n'a pas toutes les propriétés nécessaires aux souches industrielles. Une souche modèle de levure est un diploïde vrai, ou plus généralement un haploïde vrai de *Saccharomyces cerevisiae,* pouvant contenir un certain nombre de marqueurs d'auxotrophies. Les souches industrielles de levure sont en général polyploïdes.

Un développement particulier du procédé d'obtention de nouvelles souches industrielles fil après une première mise en oeuvre du procédé selon l'invention sur une souche industrielle ayant conduit à la sélection d'une souche mutée ayant le phénotype fil et portant plusieurs mutations, est le suivant :

- des ségrégeants sont isolés après sporulation dudit mutant fil issu d'une souche industrielle ;
- des ségrégeants issus dudit mutant industriel fil sont croisés avec une ou des souches haploides de laboratoire ayant le signe sexuel opposé pour sélectionner les ségrégeants issus du mutant industriel qui donnent avec la souche haploïde de laboratoire des polyploïdes ayant au moins une propriété recherchée, par exemple de résistance à un stress donné ;
- les ségrégeants ainsi sélectionnés sont croisés entre eux ;
- les polyploïdes obtenus à partir des ségrégeants issus de la souche industrielle mutée sont sélectionnés selon les critères du phénotype fil, en recherchant les souches ayant une propriété plus intéressante que la souche industrielle mutée de départ.

La présente invention a aussi pour objet de nouvelles souches eucaryotes, de préférence des nouvelles souches de levure appartenant au genre Saccharomyces et de préférence *Saccharomyces cerevisiae,* ayant le phénotype fil, susceptibles d'être obtenues par le procédé décrit ci-dessus ou une de ses variantes de réalisation. Ces souches sont de préférence des souches industrielles.

La présente invention a pour objet :

d'une part des nouvelles souches de levure de laboratoire, des nouveaux haploïdes ou ségrégeants de souches de laboratoire ou de souches industrielles présentant le phénotype fil, ces nouvelles souches étant essentiellement des souches outils ou modèles pour la construction de souches industrielles présentant le phénotype fil

d'autre part et c'est l'objet principal de l'invention des nouvelles souches industrielles de levure présentant le phénotype fil, ces nouvelles souches étant de préférence des nouvelles souches industrielles de levure de boulangerie.

Une particularité avantageuse de la présente invention est de permettre d'obtenir des souches industrielles eucaryotes fil non OGM, c'est-à-dire qui ne sont pas des organismes génétiquement modifiés au sens par exemple de la directive CEE 90/220. Compte tenu des réticences européennes non justifiées, à la lumière de la réglementation précise existant en Europe à l'égard des OGM, il est important que le procédé objet de l'invention permette la construction de nouvelles souches de levure de boulangerie industrielle, non OGM, performantes ayant le phénotype fil.

En particulier, ces nouvelles souches de levure présentant le phénotype fil donnent de préférence un taux de survie, en phase de croissance sur sucre fermentescible, d'au moins 50%, de préférence au moins 60%, encore de préférence au moins 70%, et encore plus préférentiellement au moins 75%, après traitement thermique de 20 minutes à 52°C, la phase de croissance étant définie :

- pour les souches de type laboratoire (souches haploïdes ou diploïdes vraies en général auxotrophes) et tous les ségrégeants de souches industrielles, comme une remise en culture sur sucre fermentescible (glucose) de 30 minutes à 30°C après la phase stationnaire.
- pour les souches de type industriel (souches aneuploïdes et polyploïdes) comme une remise en culture sur sucre fermentescible (glucose) de 10 minutes à 30°C après la phase stationnaire.

Les nouvelles souches de levure selon l'invention sont toutes les nouvelles souches de levure de type laboratoire (souches haploïdes ou diploïdes vraies, en général auxotrophes) et tous les ségrégeants de souches industrielles, dont la stabilité en congélation dans des pâtons contenant 20 g de farine, 15 g d'eau, 1 g de saccharose, 0,405 g de NaCl, 0,06 g de $(NH_4)_2SO_4$ et 160 mg de matière sèche de la souche considérée, définie par le ratio entre le dégagement de $CO_2$ à 30˚ après 1 mois (environ 30 jours) de conservation à -20˚C et le dégagement de $CO_2$ à 30˚C après 1 jour de conservation à -20˚C, est au moins supérieure à 60%, de préférence au moins supérieure à 70 % et de préférence encore au moins supérieure à 80%.

Les nouvelles souches de levure selon l'invention sont également toutes les nouvelles souches industrielles de levure (souches aneuploïdes ou polyploïdes) dont la stabilité en congélation en pâtons contenant 20 g de farine, 15 g d'eau, 0,405 g de NaCl, 0,06 g de $(NH_4)_2SO_4$ et 160 mg de matière sèche de la souche considérée, mesurée par le ratio entre le dégagement $CO_2$ à 30˚C après 1 mois (30 jours) de conservation à -20˚C et le dégagement de $CO_2$ à 30˚C après 1 jour de conservation à -20˚C, est au moins égale à 80%, de préférence au moins égale à 85% et de préférence encore au moins égale à 90%.

Ces tests de stabilité en congélation correspondent respectivement aux tests C2 et 1 décrits ci-après. Avant congélation à -20˚C, ces pâtons sont incubés à 30˚C pendant 30 minutes (test C1 sur levure industrielle) ou 60 minutes (test C2 sur souches de laboratoire ou sur ségrégeants).

De préférence, les nouvelles souches de levure selon l'invention permettent l'obtention de levures sèches à partir de biomasse récoltée en phase exponentielle de croissance ou en phase proche de la phase exponentielle de croissance, présentant une perte de dégagement gazeux après séchage au plus égale à 67%, de préférence au plus égale à 50% de la perte de dégagement gazeux après séchage des levures obtenues avec la souche de départ (non mutée) correspondante ou à défaut avec une souche témoin ayant les mêmes caractéristiques.

La présente invention a aussi pour objet les nouvelles souches ayant le phénotype fil :

- PVD1150 = M5 *fil1* déposée au C.N.C.M. 25 rue du Docteur Roux, F-75724 PARIS cedex, sous les n˚ I-2031 (souche contaminée) et I-2203, selon le Traité de Budapest.

- KL1 = W303 *fil2* déposée au C.N.C.M. 25 rue du Docteur Roux, F-75724 PARIS cedex, sous le n˚ I-2032, selon le Traité de Budapest.

- FD51 = HL816 *fil300* déposée au C.N.C.M. 25 rue du Docteur Roux, F-75724 PARIS cedex, sous le n˚ I-2033, selon le Traité de Budapest.

- FDH16-22 = HL822 *fil300* déposée au C.N.C.M. 25 rue du Docteur Roux, F-75724 PARIS cedex, sous le n˚ I-2034, selon le Traité de Budapest.

- AT25 = S47 *fil400* déposée au C.N.C.M. 25 rue du Docteur Roux, F-75724 PARIS cedex, sous le n˚ I-2035, selon le Traité de Budapest.

- AT28 = S47 fil 500 déposée au C.N.C.M. 25 rue du Docteur Roux, F-75724 PARIS cedex, sous le n˚ I-2036, selon le Traité de Budapest.

et toutes les nouvelles souches du même type, c'est-à-dire toutes les nouvelles souches ayant des caractéristiques similaires.

Comme le premier dépôt de la souche PVD 1150=M5 *fil1* s'est révélé être contaminé, il a été renouvelé le 20 mai 1999 sous le n˚ I-2203.

La présente invention a aussi pour objet les souches pouvant être construites par sélection de ségrégeants des souches industrielles mutées obtenues avec le phénotype fil, sur la base de croisements avec des souches haploïdes de laboratoire et étude du phénotype obtenu, puis croisement des ségrégeants sélectionnés entre eux et notamment les souches :

- AT251 déposée au C.N.C.M. 25 rue du Docteur Roux, F-75724 PARIS cedex, sous le n˚ I-2222, selon le Traité de Budapest.

- AT252 déposée au C.N.C.M. 25 rue du Docteur Roux, F-75724 PARIS cedex, sous le n˚ I-2223, selon le Traité de Budapest.

- AT254 déposée au C.N.C.M. 25 rue du Docteur Roux, F-75724 PARIS cedex, sous le n˚ I-2224, selon le Traité de Budapest.

En particulier, ledit gène est le gène ou les gènes qui confèrent le phénotype fil à l'une des souches fil qui exemplifient la présente invention et qui ont été déposées au C.N.C.M. Par exemple, ledit gène est le gène *CDC35* = *CYR1* portant une mutation conférant le phénotype fil.

Avantageusement, la dite mutation sur le gène *CDC35 = CYR1* est un changement d'une base G en une base A dans la région du gène *CDC35/CYR1* codant pour le site catalytique de l'enzyme, équivalent à un changement d'un acide aminé acide (acide glutamique) en un acide aminé basique (lysine) en position 1682 de la protéine. Cette mutation est responsable du phénotype fil de la souche PVD1150 = M5 *fil1.*

Ce gène peut aussi être le gène *YDL 035C* selon la nomenclature définie dans le projet de séquençage du génome de la levure publié dans Nature, 1992, 357, pp.38-44 ci-après appelé *GPR1* portant une mutation conférant le phénotype fil et plus particulièrement la mutation de la souche KL1 = W303 *fil2.* Ce gène peut aussi être le gène muté ou l'un des gènes mutés conférant le phénotype fil à l'une des souches C.N.C.M. I-2033, I-2035, I-2036.

Par exemple, ce gène peut coder pour une protéine associée à la protéine codée par le gène *GPR1* défini ci-dessus, et notamment ce gène peut être le gène *GPA2* de la levure portant une mutation conférant le phénotype fil.

Ladite souche industrielle de levure est avantageusement transformée de manière à ce qu'au moins certains des allèles d'un gène susceptible un fois muté de conférer le phénotype fil, portent la mutation conférant ledit phénotype fil.

L'invention concerne également l'utilisation de souches de levure mutées et sélectionnées pour leur phénotype fil par le procédé objet de l'invention ou de souches transformées de manière à leur conférer ledit phénotype, pour l'obtention de levures de panification (= levures de boulangerie) destinées aux pâtes congelées.

La présente invention a aussi pour objet l'application desdites souches mutées et sélectionnées ou transformées pour l'obtention de levures sèches de panification.

La présente invention a aussi pour objet l'application desdites souches mutées et sélectionnées ou transformées pour l'obtention de levures industrielles de brasserie, de levures industrielles de vinification, ou de levures industrielles destinées à la production d'alcool.

De manière générale, la présente invention concerne l'application des nouvelles souches eucaryotes présentant le phénotype fil dans toute condition industrielle nécessitant à la fois des propriétés de résistance au stress et de métabolisme actif desdites souches eucaryotes.

## DESCRIPTION DETAILLEE

L'invention concerne de nouvelles souches mutantes eucaryotes appelées fil, de préférence des nouvelles souches de levure fil, qui conservent une haute résistance au stress durant une phase de croissance ou de fermentation active du glucose et qui ont gardé l'essentiel de leurs propriétés métaboliques (croissance, production de métabolites primaires ou secondaires). Ces caractéristiques des souches fil étaient considérées comme inconciliables et contraires à l'équilibre biologique naturel. La recherche de telles nouvelles souches allait donc à l'encontre d'un préjugé. Le procédé nouveau employé pour l'obtention de telles souches eucaryotes aux propriétés inattendues repose sur les étapes suivantes :

- les cellules sont soumises à un traitement mutagène connu ;
- les cellules ainsi obtenues sont cultivées pour atteindre la phase stationnaire, puis elles sont remises en présence d'un sucre fermentescible comme le glucose pour être en métabolisme actif;
- lesdites cellules sont soumises à un stress important à la chaleur ou à la congélation, c'est-à-dire à un stress entraînant une forte létalité, de manière à sélectionner celles qui sont devenues résistantes au stress en phase de métabolisme actif;
- les différentes étapes successives ci-dessus ou au moins l'une d'entre elles peuvent être répétées de manière à obtenir un taux de survie par rapport à la population de départ égal ou inférieur à 1% et de préférence à 1 pour 1000, voire 1 pour 1 million ;
- les souches mutées survivantes ainsi obtenues sont testées pour vérifier leur résistance au stress à la chaleur ou à la congélation en phase de croissance ou de fermentation, de préférence elles sont également testées pour vérifier la non apparition d'une propriété secondaire gênante ou la non disparition d'une propriété secondaire utile, puis sont sélectionnées parmi les souches résistantes celles qui présentent une conservation substantielle de leurs propriétés de croissance, de fermentation, et/ou de synthèse de métabolites d'intérêt industriel et qui, de préférence, ne présentent aucune apparition d'une propriété secondaire gênante ou la disparition d'une propriété utile ;
- le maintien des propriétés correspondant au phénotype fil est enfin vérifié après un grand nombre de générations des cellules en conditions non sélectives.

Ce procédé de recherche des souches fil peut être appliqué à toute souche eucaryote, les tests étant éventuellement adaptés en fonction des caractéristiques de ladite souche eucaryote. Il est remarqué que compte tenu que les résistances sont en général croisées, les tests de résistance à la chaleur ou à la congélation sont des bons tests de sélection pour la recherche de toute nouvelle souche résistante à un stress donné, qu'elle aura à supporter lors de son utilisation industrielle. Par simplification de langage, ces deux tests sont appelés ci-après tests de résistance au choc thermique.

De manière totalement inattendue car il était admis que de telles souches n'existaient pas, le procédé ainsi décrit a conduit effectivement à la sélection de plusieurs souches parfaitement stables ayant ces caractéristiques correspondant au phénotype fil et notamment à la sélection de nouvelles souches industrielles de levures de panification (ou de boulangerie), non OGM et directement utilisables pour mettre sur le marché des nouvelles levures de panification.

Les milieux de culture utilisés dans le cadre de la réalisation de la présente invention sont:

| | | |
|---|---|---|
| Milieu YPD | extrait de levure | 10 g/l |
| | bactopeptone | 20 g/l |
| | glucose | 20 g/l |
| | | |
| Milieu YPD-A | extrait de levure | 10 g/l |
| | bactopeptone | 20 g/l |
| | glucose | 20 g/l |
| | agar | 20 g/l |
| | | |
| Milieu YP | extrait de levure | 10 g/l |
| | bactopeptone | 20 g/l |
| | | |
| Milieu SD-URA | milieu de base azoté sans acide aminé | |
| | (Yeast Nitrogen Base DIFCO®) | 6,7 g/l |
| | mélange de complémentation sans uracile | |
| | (CSM-URA, Bio 101®) | 0,77 g/l |
| | glucose | 20 g/l |
| | agar | 15 g/l |
| | | |
| Milieu R | glucose | 1,5 g/l |
| | extrait de levure | 1,0 g/l |
| | $MgSO_4$ | 0,7 g/l |
| | $CaCl_2$ | 0,4 g/l |
| | $(NH_4)_2SO_4$ | 2,0 g/l |
| | $KH_2PO_4$ | 1,87 g/l |
| | $K_2HPO_4$ | 1,1 g/l |

Les tests utilisés dans le cadre de la présente invention sont :

TESTS DE CONFIRMATION DE LA RESISTANCE AUX STRESS THERMIQUES EN PHASE DE FERMENTATION ET/OU CROISSANCE DES SOUCHES EN VUE DE LEUR SELECTION

Test T1 : Taux de survie des cellules après un choc thermique chaud

Les cellules sont cultivées sous agitation sur milieu type YPD jusqu'à l'obtention de la phase stationnaire. Ces cellules en phase stationnaire sont lavées, remises en suspension dans du milieu YP glacé, de préférence de manière à ce que la densité optique à 600 nm soit comprise entre 1 et 2 par rapport au milieu. Cette suspension est alors incubée à 30˚C jusqu'à ce qu'elle atteigne cette température. Une partie est alors conservée sur glace pour servir comme témoin des cellules en phase stationnaire tandis que l'autre partie de la suspension est l'objet d'un ajout de glucose pour obtenir une concentration finale de 100 mM, suivi d'une incubation à 30˚C de 10 à 90 minutes et d'une mise sur glace à la fin du temps choisi. Les deux suspensions sont l'objet d'une part d'un dénombrement des cellules viables après dilution de 1000 à 10000 fois et d'autre part d'un traitement thermique d'au moins 20 minutes à 52˚C suivi du même dénombrement des cellules viables. Ce dénombrement est réalisé sur YPD-A après une incubation de deux jours à 30˚C. Il peut être ainsi obtenu une mesure de résistance au stress ou taux de survie d'une part des cellules en phase stationnaire et d'autre part des cellules en métabolisme actif. Le taux de survie est exprimé par le rapport entre le nombre de colonies formées dans les échantillons traités thermiquement et le nombre de colonies formées dans les échantillons témoins.

Test T2 : Taux de survie des cellules après un choc thermique froid

Protocole identique au test T1, sauf pour le stress appliqué : la suspension est incubée de -20˚C à -30˚C pendant 1 à 12 jours.

Test T3 : Détermination de la croissance

Les cellules sont cultivées sur milieu YPD à 30˚C sous agitation 180 tours/minute jusqu'à obtention de la phase stationnaire.

La croissance, c'est-à-dire la prolifération des cellules est suivie en fonction du temps par mesure de l'absorbance (= densité optique) à 600 nm par rapport au milieu non ensemencé.

Elle est exprimée par la courbe d'évolution de l'absorbance.

Le taux de croissance est la vitesse d'augmentation du nombre de cellules, c'est-à-dire la pente de la courbe d'absorbance en fonction du temps.

Test T4 : Détermination de la croissance :

Les cellules sont cultivées sur milieu YP + glucose à 10 ou 100 mM ou sur milieu YP + mélasse de betteraves à 5g/litre à 30˚C et en plaques de microtitration.

250 microlitres de milieu sont ensemencés par des cellules en phase stationnaire de manière à obtenir 0,05 DO à 600 nm par rapport au milieu non ensemencé. Les microplaques sont agitées 30 secondes toutes les minutes et l'absorbance à 600 nm est mesurée toutes les 30 secondes. Le taux de croissance correspond à la pente de la courbe d'absorbance, le pmax ou taux de croissance maximum est alors déterminé.

Test T5 : Production sur milieu mélassé dans un temps donné :

On ensemence des boîtes contenant 100 grammes de milieu gélosé ayant la composition suivante :

| | |
|---|---|
| mélasse de betteraves | 5 g/l |
| $(NH_4)_2HPo_4$ | 0,5 g/l |
| agar | 26 g/l |
| pH | 5 - 5,5 |
| biotine ajoutée après autoclavage | 0,5$\mu$g/l |

avec l'équivalent de 2mg de matières sèches de levures par boîte. Ces boîtes sont incubées à 30˚C pendant 20 à 40 heures. On mesure la quantité finale de matière sèche levure produite en poids, sur 10 à 20 boîtes, en général sur 16 boîtes.

Pour toute souche ayant une auxotrophie, le milieu est complémenté avec le nutriment manquant.

Tests T6 et T7 de mesure de la capacité fermentative (T6) et de la perte de capacité fermentative après congélation (T7)

Pour mesurer la perte de capacité fermentative, la consommation de glucose des cellules congelées est comparée à la consommation de glucose des mêmes cellules non congelées mais stockées dans la glace. L'activité fermentative résiduelle après congélation est alors déterminée par le rapport entre la consommation de glucose des cellules congelées et la consommation de glucose des cellules non congelées. Le rapport obtenu correspond au test T7, la consommation de glucose des cellules non congelées correspond au test T6.

Les cellules sont d'abord cultivées à 30˚C sur milieu YPD jusqu'à l'obtention de la phase stationnaire puis sont resuspendues dans du milieu YP et incubées à 30˚C pendant 30 minutes. Du glucose est ajouté de manière à obtenir une concentration finale de 100 mM, et l'incubation est poursuivie à 30˚C pendant 30 minutes sans agitation et sans aération, de manière à se trouver en conditions de fermentation.

Les cellules sont ensuite resuspendues dans du milieu YP de manière à obtenir une densité optique à 600 nm égale à 15. Des échantillons de 0,03 ml sont prélevés et congelés à -25˚C pendant 1 heure dans un bain de méthanol ; ils sont ensuite stockés à -30˚C pendant 1 à 36 jours puis décongelés à température ambiante. Parallèlement, des échantillons sont stockés sur glace et servent de témoins non congelés. Les échantillons congelés et décongelés sont dilués 11 fois avec du milieu YP contenant du glucose à 10 mM, puis incubés à 30˚C. Des échantillons de 0,01 ml ne contenant plus de cellules sont prélevés après 90 et 120 minutes. Le glucose résiduel est dosé à l'aide du kit Trinder Reagent[®] (fournisseur SIGMA) par ajout de 0,2 ml de réactif. Après 15 minutes d'incubation à 30˚C, l'absorbance est mesurée à 505 nm. Il a été vérifié que, dans ces conditions, il existait une corrélation linéaire entre la quantité de cellules actives et la consommation de glucose, ce qui rend aisé la détermination de l'activité fermentative résiduelle après congélation.

TESTS DE DETERMINATION DE LA FORCE FERMENTATIVE DES LEVURES EN VUE DE LEUR SELECTION

Les tests A1, A20, A'1, A'20, C1 et C2, utilisés pour déterminer la force fermentative les levures c'est-à-dire leur capacité à produire du $CO_2$ sont réalisés à l'aide du fermentomètre de Burrows et Harrison, décrit dans le " Journal of the Institute of Brewing", 1959, LXV, 1, janvier-février, et sont exactement définis de la manière suivante :

Test A1 : force fermentative (levures fraîches, souches industrielles)

A 20 g de farine incubée à 30˚C, on ajoute un poids de levure correspondant à 160 mg de matières sèches, cette levure étant délayée dans 15 ml d'eau contenant 27 g de NaCl par litre et 4 g de $(NH_4)_2SO_4$ par litre ; on malaxe à l'aide d'une spatule pendant 40 secondes, de manière à obtenir une pâte que l'on place à 30˚C ; treize minutes après le début du malaxage, le récipient contenant la pâte est hermétiquement fermé ; la quantité totale de dioxyde de carbone ($CO_2$) produit est mesurée après 60 et 120 minutes, cette quantité est exprimée en ml à 20˚C et sous 760 mm de mercure (Hg).

Test A20 : force fermentative (levures fraîches , autres souches)

Test identique au test A1, mais la composition des pâtons est modifiée comme suit : 1g (un gramme) de saccharose est ajouté au mélange composé de farine, d'eau, de levure et de sel, avant d'effectuer le pétrissage. De plus, le dégagement de $CO_2$ est mesuré sur 120 et 240 minutes à 30˚C (au lieu de 60 et 120 minutes pour le test A1).

Test A'1 : force fermentative (levures sèches souches industrielles)

Test identique au test A1, mais préalablement au malaxage, on réhydrate en 15 minutes les 160 mg de matières sèches de la levure, qui se présente sous forme de levure sèche dans de l'eau distillée, à 20˚C ou à 38˚C ; on utilise à cet effet 40% du volume d'eau d'hydratation mis en oeuvre ; le complément en eau, additionné de 405 mg de NaCl, est ajouté à l'issue des 15 minutes de réhydratation.

Test A'20 : force fermentative (levures sèches autres souches)

Test identique au test A'1, mais on ajoute à la farine 1 g de saccharose ; la quantité totale de gaz produit est par ailleurs mesurée sur 240 minutes.

Test C1 : force fermentative (souches industrielles après congélation)

Test identique au test A1 mais il faut façonner au moins six pâtons par souche. Les pâtons sont façonnés selon les conditions du test A1, mais après pétrissage, les pâtons sont incubés à 30˚C pendant 30 minutes. Les pâtons pré-fermentés sont ensuite immédiatement stockés à -20˚C et conservés à cette température sur des durées allant de 1 jour à 2 mois. Pour mesurer l'activité fermentative après conservation à -20˚C, le pâton congelé est placé dans une enceinte à 30˚C ; après treize minutes, le récipient contenant le pâton est fermé hermétiquement et la quantité totale de $CO_2$ produite (exprimée en ml à 20˚C et sous 760 mm de Hg) est mesurée sur 120 minutes.
Pour une souche donnée, le dégagement de $CO_2$ de référence correspond au dégagement de $CO_2$ du pâton conservé pendant un seul jour à -20˚C. Les autres pâtons sont décongelés à intervalles réguliers (par exemple, après 1 semaine, 2 semaines, 1 mois, 1 mois ½ et 2 mois de conservation à -20˚C), et le dégagement de $CO_2$ mesuré de manière à suivre l'évolution de l'activité fermentative en fonction de la durée de conservation à -20˚C.
La stabilité en congélation est le rapport entre le dégagement de $CO_2$ sur 2 heures après 1 mois (30 jours) de congélation et le dégagement gazeux de référence après 1 jour de congélation.

Test C2 (autres souches après congélation)

Test basé sur le test C1 avec les modifications suivantes :

- 1 g de saccharose est ajouté au mélange farine-eau-sels-levure avant pétrissage,
- les pâtons sont pré-fermentés 60 minutes à 30˚C avant congélation à -20˚C,

- le dégagement de $CO_2$ est mesuré sur 240 minutes à 30˚C.

Test R : détermination de l'assimilation d'éthanol

100 ml de milieu R sont ensemencés de manière à ce que la densité optique du milieu ensemencé soit de 0,05 DO à 600 nm par des cellules en phase stationnaire, obtenues après culture sur milieu YP + glucose à 100 mM pendant 24 à 48 heures. Le milieu R ensemencé est alors placé à 30˚C et agité en continu à 180 tours par minute. L'absorbance à 600 nm est suivie par des prélèvements réguliers jusqu'à l'obtention d'une DO stable. Dans ces conditions, une souche n'assimilant pas l'éthanol atteint une DO environ 4 fois plus faible qu'une souche assimilant normalement l'éthanol. Seules sont retenues dans ce test les souches atteignant une DO au moins égale à 50% de celle de la souche témoin (en général la souche non mutée) et de préférence au moins 80%, et encore de préférence au moins 90%.

L'ensemble des tests ainsi décrits pour la mise en oeuvre de l'invention sont des tests de nature biologique et leur reproductibilité d'un laboratoire à un autre pose souvent des problèmes délicats. En conséquence, ils doivent le plus souvent être interprétés de manière relative par rapport à un témoin. La mise au point des tests doit être menée de manière à retrouver les valeurs indiquées pour le témoin ou, de préférence, les témoins de manière à avoir une échelle de valeurs reproductible. Cette mise au point des tests doit être soignée et minutieuse.

Il est clair pour l'homme de l'art que chaque étape du procédé devra être adaptée en fonction des caractéristiques des souches utilisées et de leur létalité ou taux de survie dans les différents tests. Les indications données ci-après sur la mise en oeuvre des différentes étapes du procédé objet de l'invention ne sont que des exemples dans le cadre de l'application du procédé à *Saccharomyces cerevisiae.*

Le procédé objet de l'invention de recherche de nouvelles souches à la fois métaboliquement actives et résistantes au stress dont le principe est donné ci-dessus peut être mis en oeuvre :

- sur des souches de laboratoire, c'est-à-dire sur des souches modèles dont l'ensemble des caractéristiques génétiques sont bien connues et contenant en général des marqueurs d'auxotrophie ;
- sur des souches industrielles, en général beaucoup plus complexes au plan génétique, du fait qu'elles ne sont pas des haploïdes ou des diploïdes vrais contrairement aux souches de laboratoire, et qui ont été sélectionnées pour leurs performances industrielles. Parmi les souches industrielles on peut distinguer :

  • les souches industrielles dans leur état le plus stable, en général sous forme polyploïde,
  • les ségrégeants de souches industrielles, c'est-à-dire les formes sexuées qui sont en général moins stables, mais sont utilisables pour des constructions de génétique classique.

Dans le cadre de recherches ayant pour but de caractériser les mutations conférant le phénotype fil, le nouveau procédé objet de l'invention sera de préférence mis en oeuvre sur des souches de laboratoires, de préférence haploïdes.

La caractérisation du ou des gènes concernés sera entreprise, ce qui permettra ensuite de construire des souches industrielles.

Dans le cadre de recherches ayant pour but d'obtenir directement des souches industrielles, le procédé sera mis en oeuvre directement soit sur des ségrégeants de souches industrielles, soit directement sur les souches industrielles. L'emploi de ségrégeants, signifie que les ségrégeants fil obtenus pourront et devront faire l'objet de constructions par génétique classique pour retrouver toutes les caractéristiques désirées des souches industrielles, en plus du phénotype fil.

L'obtention de souches industrielles fil, non OGM, c'est-à-dire qui ne sont pas des organismes génétiquement modifiés est l'objet préféré de l'invention.

Les caractéristiques essentielles de ces souches industrielles sont les suivantes :

- conservation de l'essentiel des propriétés des souches industrielles dont elles sont dérivées et qui sont effectivement utilisées en raison de leur performance
- résistance significativement augmentée aux conditions de stress rencontrés dans les processus de fabrication industrielle ou artisanale dans lesquels elles sont utilisées
- progrès ou meilleur résultat du fait du phénotype fil.

Compte tenu de sa définition intrinsèque, le phénotype fil ne peut pas correspondre pour une levure par exemple :

- à une délétion des gènes *RAS1* ou *RAS2*, compte tenu de la très mauvaise utilisation de l'éthanol qu'une telle délétion implique (J.F. Cannon et al, Genetics, 113, p.250, June 1986). Les souches fil doivent montrer qu'elles sont capables de réassimiler une partie de l'alcool dans le test R car c'est une propriété secondaire utile qui ne doit pas disparaître.

- aux mutants cyrl-T1 et cyr2-T2 décrits par Iida (Mol.Cell.Biol., Dec. 1988, pp. 5555-5560) compte tenu des bas taux de croissance de ces mutants.
- à une sensibilité particulière à la température observée lors de sa multiplication ou de sa fermentation.
- à une simple délétion d'un ou plusieurs gènes codant pour une des tréhalases de la levure, la non synthèse ou la faible synthèse de tréhalases en condition de croissance étant un facteur intéressant comme facteur secondaire de la résistance au stress, insuffisant à lui seul.
- à une transformation avec de l'ADN codant pour une protéine à activité SOD (SuperOxyde Dismutase) et avec de l'ADN codant pour une protéine à activité catalase, car cela correspond à des facteurs d'ordre secondaire et d'action limitée, et à des pertes de performances liées à l'expression de ces gènes dans le cadre de l'utilisation normale desdites levures.

Une souche de levure de panification qui après mutation donnerait par exemple une mauvaise odeur aux pains, un mauvais goût aux pains ou un goût anormal, de fait d'un métabolisme secondaire touché par la mutation, ne serait pas une souche correspondant au phénotype fil, car selon la définition préférentielle qui lui est donnée dans la présente invention, le phénotype fil correspond à une levure n'ayant aucune propriété secondaire gênante pour son emploi.

De manière avantageuse, les traitements mutagènes employés dans le procédé selon l'invention sont les suivants. Les cellules de levure *Saccharomyces cerevisiae* sont cultivées sur un milieu YPD puis subissent un traitement mutagène par un agent chimique tel que l'EMS (Ethyl-Methyl-Sulfonate) ou par ultraviolets selon des protocoles classiques (Sherman et al., 1986, Cold Spring Harbor Lab Press ; Spencer et al., 1988, Yeast a practical approach, Ed. Campbell et Duffus). Les conditions de la mutagenèse sont en général choisies pour obtenir un taux de survie des cellules de l'ordre de 1 à 20%, de préférence environ 10%. Les cellules sont alors lavées, remises en suspension dans un milieu YPD et cultivées jusqu'à atteindre la phase stationnaire. Puis une fraction connue de la culture est prélevée, éventuellement lavée, transférée sur milieu YPD et incubé à 30˚C, pendant 30 à 90 minutes. Une variante peut consister à ajouter à la culture en phase stationnaire 100 mM de glucose et à l'incuber à 30˚C pendant 30 à 90 minutes.

La culture est ensuite soumise soit à un choc thermique chaud par incubation entre 52 et 65˚C, de préférence à 56˚C pendant 30 minutes ou plus, soit à un choc thermique froid (congélation) par incubation entre -20˚C et -40˚C pendant 1 à 3 jours. La congélation ne provoquant qu'une légère perte de viabilité des cellules de levure, le traitement est répété jusqu'à 200 fois, jusqu'à l'obtention d'un taux de survie des cellules inférieur à 1 pour 10000, de préférence inférieur à 1 pour 100000 et de préférence encore inférieur à 1 pour 1000000.

Dans une forme de réalisation particulière du protocole de sélection, les cellules après mutagenèse sont introduites à proportion de $4.10^8$ cellules/g de pâton dans des petits pâtons d'environ 0,5 g, composés d'eau (42,5%), de farine (56,5%), de NaCl (1%) et fermentés 30 minutes à 30˚C de manière à ce que les cellules de levure quittent la phase stationnaire. Les pâtons sont alors successivement congelés à -30˚C et décongelés à température ambiante, jusqu'à 200 fois de manière à ce que seulement quelques centaines ou quelques milliers de cellules survivent.

A ce stade, les cellules ayant survécu au choc thermique (chaleur ou congélation) sont des mutants résistants au stress dont il convient de vérifier d'une part la persistance de cette propriété durant la fermentation ou la croissance sur plusieurs générations, et d'autre part le maintien de leur capacité de croissance et de production de métabolites. Pour vérifier la résistance en phase de croissance et/ou de fermentation, les taux de survie sont mesurés selon les tests T1 et/ou T2, et/ou des tests équivalents. Les souches mutées sont sélectionnées sur l'augmentation de leur taux de survie dans un stress important en phase de fermentation et/ou de croissance comme ceux des tests T1 et T2 par rapport à leur souche de départ, et dans ce cas là on recherchera de manière générale un taux de survie au moins 1,5 fois plus élevé. Les souches mutées peuvent aussi être sélectionnées sur la comparaison de leur taux de survie en phase de croissance et/ou de fermentation avec leur taux de survie en phase stationnaire, ou avec le taux de survie en phase stationnaire de la souche de départ.

De préférence les souches mutées sélectionnées auront après une phase de croissance et/ou fermentation comme celle définie dans les tests T1 ou T2 au moins 50%, de préférence au moins 60%, de préférence encore au moins 70%, et encore plus préférentiellement au moins 80% de leur taux de survie en phase stationnaire.

Une variante consiste à mesurer la résistance des souches mutées obtenues, en congélation selon les tests C1 ou C2 ou des tests équivalents, ou encore à mesurer la résistance au séchage en utilisant des rapports à partir des valeurs obtenues dans les tests A et A' pour la levure avant et après séchage.

Pour vérifier la conservation des propriétés de croissance et de fermentation, il peut être effectué une comparaison entre la souche de départ et la souche mutée présentant un phénotype de résistance au stress en phase métabolique active, dans un des tests T3, T4 ou T5 et dans un des tests A ou dans des tests similaires permettant de mesurer les propriétés d'intérêt des souches considérées. Une conservation d'au moins 80% de ces propriétés est nécessaire pour correspondre au phénotype fil.

De préférence, il sera vérifié que la mutation n'a entraîné aucune perte d'un caractère secondaire intéressant.

Par exemple, il sera vérifié à l'aide du test R qu'elle a une assimilation suffisante de l'alcool, et qu'elle donne dans ce test une Densité Optique au moins égale à 50% de celle de la souche témoin, de préférence au moins 80% et de préférence encore au moins 90%. Il sera également vérifié qu'il n'y a production d'aucun métabolite inopportun. Par exemple, il sera vérifié sur des souches industrielles de levure de boulangerie que dans les conditions habituelles de panification notamment rencontrées en Europe, aux USA, il n'y a aucun mauvais goût ou mauvaise odeur conféré aux pains.

De plus il est indispensable de vérifier que la mutation est stable, c'est-à-dire que le phénotype fil est stable. En conséquence, l'ensemble des vérifications définies ci-dessus doivent être répétées sur la souche mutée réisolée après un grand nombre de multiplications en milieu non sélectif, comme par exemple après 10 cultures successives de 2 jours en milieu YPD-A à 30˚C ou encore 10 cultures en milieu YPD à 30˚C et ensemencement au centième à partir de la culture précédente.

Les souches ayant traversé avec succès ces différents filtres de sélection sont les souches parfaitement stables dotées du phénotype fil selon l'invention. Elles sont résistantes au(x) stress en phase de métabolisme actif, elles ont conservé une croissance et un pouvoir fermentatif intéressants.

Les souches fil sont destinées à être utilisées dans des applications industrielles particulièrement exigeantes en ce qui concerne la résistance au stress en phase de métabolisme actif. Des propriétés recherchées pour les souches fil sont par exemple une stabilité élevée en congélation et/ou au séchage. Par stabilité, on entend que le pouvoir fermentatif de la levure ayant été placée dans des conditions agressives conserve un niveau élevé par rapport au pouvoir fermentatif de la même levure avant le traitement agressif, notamment le séchage et la congélation en pâtons.

La stabilité de la levure en pâtons congelés ou surgelés est un critère important en boulangerie industrielle où les pâtons sont congelés alors que la fermentation a déjà commencé, et que la durée de congélation peut aller de quelques jours à plusieurs semaines. Il est crucial que lors de la décongélation la levure n'ait pas perdu l'essentiel de son pouvoir fermentatif.

Une souche de levure industrielle ayant d'une part des propriétés équivalentes aux souches industrielles actuellement commercialisées en France ou en Europe et d'autre part ayant une stabilité en congélation en pâtons selon le test C1 au moins égale à 80%, de préférence au moins égale à 85% et de préférence encore au moins égale à 90%, représente un progrès important pour la fabrication des pâtes congelées. De préférence, cette souche industrielle de levure de panification est non OGM.

Au niveau de la sélection de mutants fil à partir de souches de laboratoire pour ensuite caractériser le ou les gènes à l'origine de la mutation, ou de ségrégeants devant servir à la construction de nouvelles souches industrielles par génétique classique, l'expérience a montré qu'il était nécessaire d'adopter au niveau de la sélection des taux de stabilité moindre en pâte congelée, ces souches étant en général par nature moins résistantes au stress d'une congélation.

La levure de boulangerie, sous forme sèche à au moins 92% de matières sèches, de préférence au moins 94% de matières sèches, doit conserver ses performances fermentatives, à matières sèches égales, malgré le stress lié à la déshydratation.

Il est bien connu que les levures ont une perte d'activité au séchage d'autant plus faible qu'elles sont récoltées dans une phase de croissance faible, c'est-à-dire dans des conditions assez éloignées de celles dans lesquelles elles ont leur potentiel fermentaire maximal. Des souches fil permettent de déplacer cet équilibre. Cette possibilité peut être mesurée en comparant la perte d'activité au séchage de la souche fil par rapport à la perte d'activité au séchage de la souche de départ ou d'une souche témoin cultivée dans des mêmes conditions de phase de croissance active. La perte d'activité au séchage est définie selon la formule :

$$100 - \frac{A'}{A} \times 100$$

A' est la force fermentative mesurée pour la levure sèche selon un test A', A est la force fermentative mesurée pour la levure avant séchage selon le test A correspondant.

Le procédé selon l'invention a conduit à l'obtention de deux types de mutants de souches de levure de laboratoire appelés *fil1* et *fil2,* une numérotation différente (*fil1*, *fil2*,...) étant donnée pour chaque mutation semblant affecter un gène différent.

La première famille de mutants est la famille *fil1* dont la souche type est la souche PVD1150 = M5 *fil1*. Ce mutant est dérivé de la souche de laboratoire de type sauvage M5 (Schaaff et al., 1989, Curr. Genet., 15, pp.75-81). La mutation *fil1* est portée par le gène *CYR1/CDC35,* qui code pour l'adénylate cyclase, c'est-à-dire pour l'enzyme qui synthétise l'AMPc. La mutation a été identifiée comme étant une substitution d'un résidu glutamate en un résidu lysine en position 1682 de la protéine. Ce changement ponctuel est localisé dans le domaine catalytique de l'adénylate cyclase, à proximité de la région qui est considérée comme impliquée dans l'activation de l'adénylate cyclase par les protéines Ras. Le changement d'un acide aminé acide en un acide aminé basique est susceptible d'influencer fortement l'activité du site catalytique, ce d'autant plus que ce changement intervient dans une zone très conservée. Cette mutation *fil1* introduite dans le gène *CYR1/CDC35* de 2 souches différentes de laboratoire, leur a fait acquérir ledit phénotype fil. En conséquence la mutation *fil1* dans le gène *CYR1/CDC35* permet de construire des souches industrielles ayant le phénotype fil.

La seconde famille de mutants est la famille *fil2* dont la souche type est la souche KL1 = W303 *fil2.* Ce mutant est dérivé de la souche de laboratoire W303-1A (Thomas et Rothstein (1989) Cell, 56, pp.619-630).

La mutation *fil2* est portée par le gène *YDL035c* appelée gène *GPR1* par Xue et al, EMBO J., 1998, 17,7, 1996-2007. Le gène *GPR1* a été isolé et identifié comme étant un gène codant pour une protéine s'associant à la protéine Gpa2 codée par le gène *GPA2*. L'introduction de ce gène *GPR1* muté dans les souches industrielles permettra de leur conférer le phénotype fil.

Des mutations sur la famille des gènes codant pour des protéines associées à la protéine codée par le gène *GPR1,* comme une mutation sur le gène *GPA2*, sont susceptibles avec une très forte probabilité de conduire au phénotype fil.

Le procédé objet de l'invention a conduit à l'obtention de deux ségrégeants industriels portant la mutation appelée *fil300*.

Ce sont les souches FD51 = HL816 *fil300* et FDH16-22 = HL822 *fi1300*. Ces deux souches sont dérivées d'un ségrégeant industriel. Elles permettent par croisement avec d'autres ségrégeants industriels, puis sélection d'au moins deux ségrégeants de signe opposé portant le phénotype fil, et enfin croisement entre ces ségrégeants, de construire de nouvelles souches industrielles ayant le phénotype fil

Plusieurs mutants ont été obtenus par le procédé objet de l'invention à partir de la souche industrielle polyploïde S47 qui est déposée au C.N.C.M. sous le n˚ I-2037. Cette souche a été choisie car elle est la souche la plus utilisée en France actuellement pour la panification des pâtes crues congelées.

Ces mutants sont les souches :

    AT25 = S47 *fil400*
    AT26
    AT28 = S47 *fil500*
    AT31

Les deux souches AT25 et AT28 ont fait l'objet d'études complètes conduisant à la conclusion qu'il s'agit a priori de deux mutations différentes. La souche AT25 permet une utilisation directe comme souche industrielle de levure de panification pour l'application pâtes congelées.

De manière générale, l'invention n'est pas limitée à l'isolement des gènes portant la mutation dans les souches de laboratoire fil. Elle englobe la même démarche pour isoler le gène portant la mutation dans les ségrégeants industriels ou dans les souches industrielles. Dans les souches industrielles polyploïdes, la mutation est probablement dominante et en conséquence la stratégie d'isolement du ou des gènes concernés devra être adaptée en conséquence. Dans cette hypothèse, on construit une banque d'ADN génomique du mutant fil dans un vecteur centromérique comme le vecteur Ycp50, usuellement disponible, qui possède le marqueur *URA3*. On transforme une souche de laboratoire de levure auxotrophe pour l'uracile avec cette banque d'ADN et on recherche parmi les transformants qui n'ont plus besoin d'uracile, ceux qui ont acquis le phénotype fil, par la technique de mise en évidence de mutants fil décrite précédemment.

Il est remarquable que les différents mutants fil obtenus n'ont pas des mutations qui affectent la même voie métabolique. Cela est montré par le tableau suivant :

| Propriétés | Souches | | | |
| --- | --- | --- | --- | --- |
| | *fil1* | *fil2* | *fil300* | AT25 ; AT26 ; AT28 ; AT31 |
| Réduction de la perte de résistance au stress induite par la fermentation | oui | oui | oui | oui |
| Déficience du signal AMPc induit par le glucose | oui | oui | oui | non |
| Déficience de l'accumulation d'AMPc induite par l'acidification | oui partiellement | non | n.d. | n.d. |
| Niveau de tréhalose accru | oui | oui | oui | oui |
| Phase de latence normale pour culture sur glucose | oui | oui | oui | oui |
| Taux de croissance normal sur glucose | oui | oui | oui | oui |

(suite)

| Propriétés | Souches | | | |
|---|---|---|---|---|
| | *fil1* | *fil2* | *fil300* | AT25 ; AT26 ; AT28 ; AT31 |
| Production de $CO_2$ normale dans la pâte | oui | oui | oui | oui |
| Récolte normale sur mélasse | oui | oui | oui | oui |

L'augmentation de la teneur en AMPc induite par le glucose est affectée dans les mutants *fil1, fil2* et *fil300.* Elle ne l'est pas en revanche dans les mutants *fil400* (AT25), *fil500* (AT28), et dans les mutants AT26 et AT31. Des ségrégeants issus de AT25 peuvent cependant montrer une déficience dans cette augmentation de la teneur en APMc induite par le glucose, contrairement à la souche AT25. C'est une présomption que la souche AT25 comporte plusieurs mutations différentes.

Dans le cas des mutants *fil1*, *fil2* et *fil300,* les mutations affectent la voie Ras-AMPc-PKA. Cela est totalement normal pour la mutation *fil1.* Cela conduit à dire que le gène *GPR1* dont la fonction est mal connue a une action sur cette voie.

Dans le cas des autres mutants, la mutation ne semble pas concerner à titre principal la voie Ras-AMPc-PKA.

Il est connu que la voie Ras-AMPc-PKA n'est pas la seule voie impliquée dans les mécanismes de résistance au stress. Le nouveau procédé selon l'invention est un outil particulièrement intéressant pour :

- isoler des nouveaux mutants résistants au stress
- caractériser le ou les gènes concernés dans différentes voies métaboliques
- construire à l'aide de ces gènes des nouvelles souches industrielles particulièrement performantes.

Les résistances au stress sont souvent croisées. Les mêmes voies métaboliques se retrouvent souvent chez de nombreux eucaryotes différents. Notamment des voies équivalentes à la voie Ras-AMPc-PKA ont été décrites chez de nombreux eucaryotes. En conséquence, la présente invention ne se limite pas à l'obtention de levures fraîches ou sèches de panification, de brasserie, de vinification, de distillation, pour la production de protéines hétérologues, mais à l'obtention de toute nouvelle souche eucaryote d'intérêt pour tout usage industriel, comme la production d'acides organiques, d'acides aminés, d'enzymes, etc...

Si des présomptions indiquent que le procédé objet de l'invention a conduit à la sélection de mutants industriels fil portant des mutations sur plusieurs gènes différents, affectant éventuellement plusieurs voies métaboliques, il est alors intéressant de sélectionner des ségrégeants issus de ce mutant industriel fil et portant des propriétés dominantes intéressantes par croisement avec un haploïde de laboratoire et étude du polyploïde obtenu, puis de croiser les ségrégeants sélectionnés pour leur apport de propriétés dominantes entre eux, et de sélectionner les nouvelles souches ainsi créées pour leur(s) résistance(s) au stress et leurs propriétés en métabolisme actif, et de rechercher ainsi des souches ayant au moins une propriété plus intéressante que le mutant industriel de départ.

La présente invention est également illustrée par les exemples suivants. La liste des figures venant à l'appui de ces exemples est donnée ci-après. Les figures sont désignées par la nomenclature suivante. Le premier chiffre est le numéro de l'exemple dans lequel la figure est décrite, le second chiffre est son ordre dans ledit exemple.

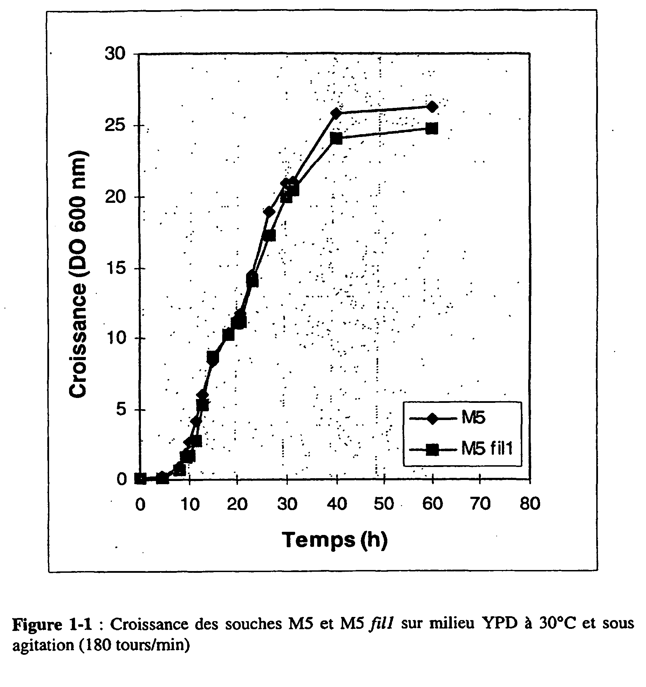

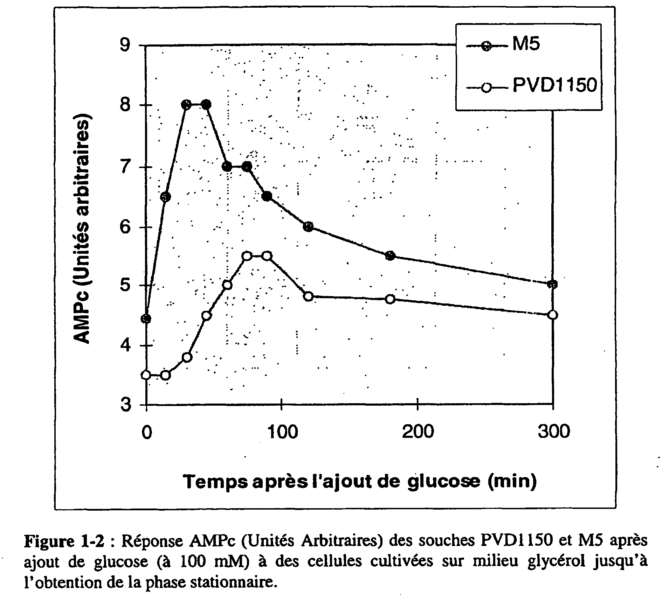

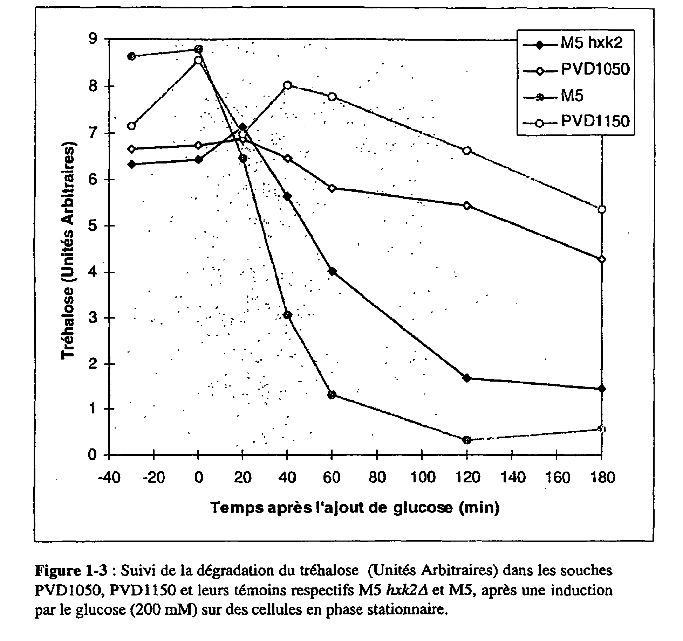

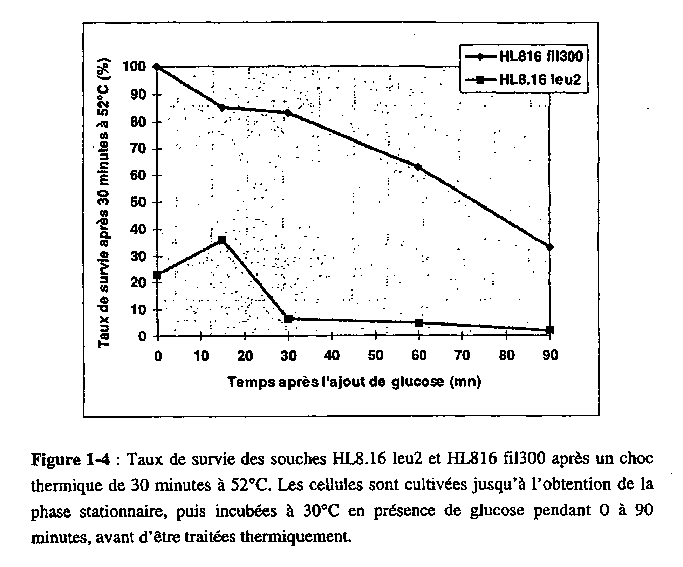

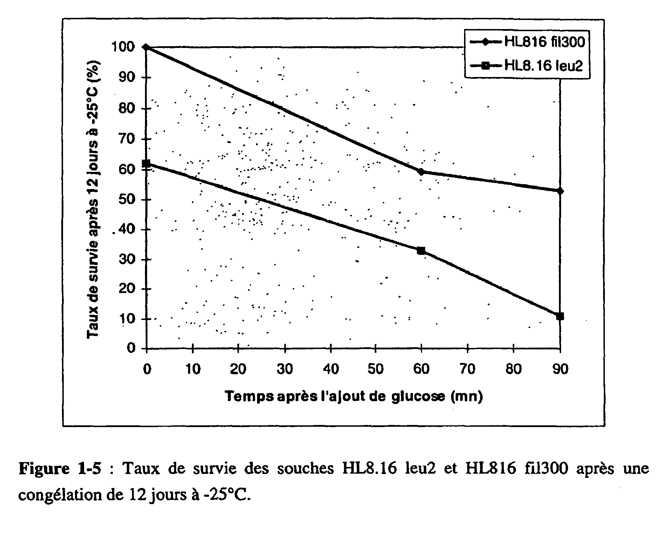

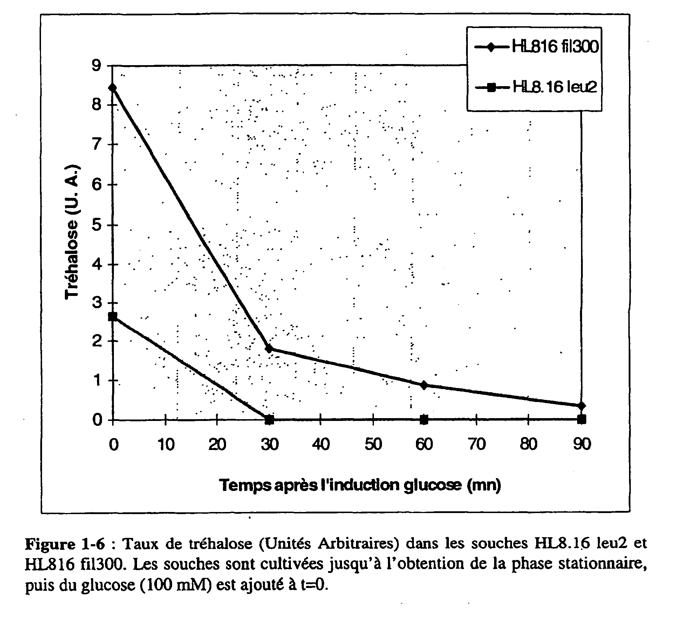

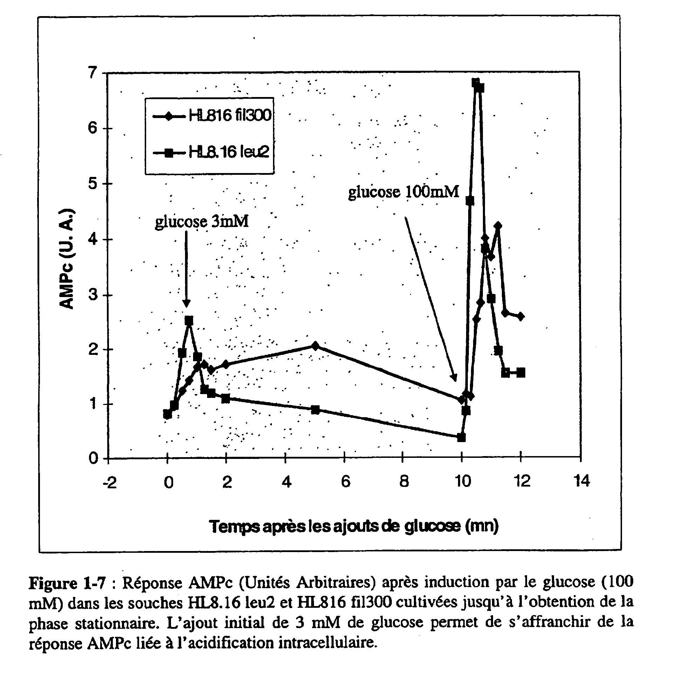

**Figure 1-1** : Croissance des souches M5 et M5 *fil1* = PVD1150 sur milieu YPD à 30˚C et sous agitation (180 tours/min) selon le test T3.

**Figure 1-2 :** Réponse AMPc (Unités Arbitraires) des souches PVD1150 et M5 après ajout de glucose (à 100 mM) à des cellules cultivées sur milieu glycérol jusqu'à l'obtention de la phase stationnaire.

**Figure 1-3 :** Suivi de la dégradation du tréhalose (Unités Arbitraires) dans les souches PVD1050, PVD1150 et leurs témoins respectifs M5 *hxk2*Δ et M5, après une induction par le glucose (200 mM) sur des cellules en phase stationnaire.

**Figure 1-4 :** Taux de survie des souches HL8.16 *leu2* et HL816 *fil300* après un choc thermique de 30 minutes à 52˚C selon le test T1.

**Figure 1-5 :** Taux de survie des souches HL8.16 *leu2* et HL816 *fil300* après une congélation de 12 jours à -20˚C selon le test T2.

**Figure 1-6 :** Taux de tréhalose (Unités Arbitraires) dans les souches HL8.16 *leu2* et HL816 *fil300.* Les souches sont cultivées jusqu'à l'obtention de la phase stationnaire, puis du glucose (100 mM) est ajouté à t=0.

**Figure 1-7 :** Réponse AMPc (Unités Arbitraires) après induction par le glucose (100 mM) dans les souches HL8.16 *leu2* et HL816 *fil300* cultivées jusqu'à l'obtention de la phase stationnaire. L'ajout initial de 3 mM de glucose permet de s'affranchir de la réponse AMPc liée à l'acidification intracellulaire.

**Figure 2-1** : Suivi de la dégradation de tréhalose (Unités Arbitraires) après une induction par le glucose à t = 0 (sur des cellules en phase stationnaire).

**Figure 2-2** : Suivi de la réponse AMPc (Unités Arbitraires) après une induction (à t = 0) par le glucose sur des cellules en phase exponentielle de croissance sur maltose. a) témoin S47 et mutant AT25 ; b) témoin S47 et mutants AT26, AT28 et AT31.

**Figure 3-1 :** Stratégie de gap-filling (remplissage de l'ADN manquant) utilisée pour l'isolement du gène portant la mutation *fil1* dans la souche PVD1150.

**Figure 3-2 :** Carte physique du vecteur pUC18-CYR1mut -URA3 [Sn].

**Figure 6-1 :** Stabilité en congélation mesurée par le ratio entre le dégagement de $CO_2$ au jour de mesure et le dégagement de $CO_2$ après 1 jour de conservation à -20˚C.

**EXEMPLE 1 : Utilisation du stress thermique comme outil d'isolement de mutants fil**

**A. Obtention des souches ayant le phénotype *fil1***

**a) Obtention des souches PVD1050 et PVD1150**

La souche de départ est la souche haploïde M5 *hxk2Δ* (souche portant une délétion du gène de l'hexokinase II) qui est issue de la souche haploïde M5, elle-même issue de la souche diploïde M5 (Schaaff et coll. (1989), Curr. Genet. 15: 75-81). Une mutagenèse à l'E.M.S. (Ethyl Méthyl Sulfonate) a été réalisée selon la technique décrite dans " Methods in yeast Genetics, Cold Spring Harbor Laboratory Press " (Sherman et coll., 1986) de manière à obtenir un taux de survie d'environ 10%. Après ce traitement, les cellules ont été lavées et remises en suspension dans du milieu YPD. Puis elles ont été cultivées jusqu'à l'obtention de la phase stationnaire. Une prise de 0,25 ml de cette culture a alors été prélevée et utilisée pour inoculer 25 ml de milieu YPD. Le milieu inoculé a été incubé à 30˚C pendant 90 minutes, puis transféré à 52˚C pendant 30 minutes. Il a ensuite été incubé à 30˚C pendant 24 heures, puis 0.5 ml de la culture a été transféré dans 25 ml de milieu YPD. Ce milieu a été incubé à 30˚C pendant 90 minutes. Des fractions de 100 μl ont ensuite été prélevées et placées à 56˚C pendant 30 minutes. Ensuite, les cellules survivantes ont été étalées sur milieu YPD-A. La résistance au stress a alors été évaluée sur les cellules s'étant développées.

Pour tester cette résistance, les cellules de levure des souches obtenues ont été testées selon le test T1, avec une incubation à 52˚C fixée à 30 minutes. Dans les conditions de ce test, et après vérification de la conservation des propriétés de croissance et de fermentation, et la stabilité de la mutation, une souche a été sélectionnée et nommée Mutl ou encore PVD1050. Il a été démontré que cette souche porte une mutation stable, qui a été nommée *fil1* et qui est monogénique et récessive. La souche haploïde PVD1050 présente un taux de survie après traitement thermique qui est étonnamment élevé, et cela en phase stationnaire comme en phase exponentielle, comme le montrent les résultats du tableau 1-A. Il est surprenant, pour une souche de *Saccharomyces cerevisiae*, de conserver en phase exponentielle près de 100% de viabilité après un traitement thermique de 30 minutes à 52˚C. Il est également surprenant de constater que cette souche PVD1050 n'est pas significativement pénalisée en croissance et en fermentation par rapport à la souche M5 *hxk2Δ*, étant entendu que la délétion du gène *HXK2* pénalise quant à elle, entre autres, la croissance.

Afin de s'affranchir de cette délétion du gène *HXK2,* la souche haploïde PVD1050 (=M5 *hxk2Δ fil1*) a été croisée avec la souche M5 haploïde, puis il a été recherché dans la descendance une souche portant la mutation *fil1* mais possédant un gène *HXK2* sauvage. La souche haploïde PVD1150 encore appelée souche M5 *fil1* a ainsi été isolée. Cette souche présente le même phénotype de thermorésistance que la souche PVD1050, c'est-à-dire un taux de survie extrêmement élevé dans le test T1 (tableau 1-A).

**Tableau 1-A -** Evaluation du taux de survie des souches *fil1* et de leurs témoins après un choc thermique de 30 minutes à 52˚C.

| Test T1 avec taux de survie après 30' à 52˚C (%) | | | | |
|---|---|---|---|---|
| | **En Phase stationnaire** | **En Métabolisme actif** = incubation glucose de : | | |
| | | 30' | 60' | 90' |
| M5 *hxk2Δ* | 70 % | 40% | 30 % | 15 % |
| PVD 1050 | 100 % | 98% | 98 % | 98 % |
| M5 | 50 % | 20 % | 10% | 5% |
| PVD 1150 | 99% | 96 % | - | - |

**b) Etude de la souche PVD1150**

L'étude des caractéristiques associées à la mutation *fil1* a été réalisée sur cette nouvelle souche haploïde M5 *fil1* en prenant la souche haploïde M5 comme témoin.

Dans un premier temps, la croissance sur glucose de la souche PVD1150 est comparée à celle du témoin M5 de manière à étudier l'influence éventuelle de la mutation *fil1* sur la croissance. Les souches ont été cultivées sur milieu YPD selon les conditions du test T3. Les résultats (figure 1-1) montrent clairement que la mutation *fil1* affecte très peu la croissance de la souche M5. Ces mêmes souches ont ensuite été cultivées sur milieu mélasse gélosé selon les conditions du test T5. Un rendement de croissance identique a été obtenu pour les deux souches (tableau 1-B).

Les levures récoltées ont été utilisées pour réaliser une mesure de force fermentative dans les conditions du test A20. Ce test montre que la perte de force associée à la mutation *fil1* ne dépasse pas 20% (tableau 1-B).

**Tableau 1-B**

|  | Test T5 <br> Récolte (en matière sèche de levure) <br> après 40 h [pour 8 g de mélasse] | Test A20 <br> Force fermentative <br> (ml $CO_2$ dégagé en 2 heures) |
|---|---|---|
| M5 | 1.70 g | 66-75 ml |
| M5 *fil1* | 1.74 g | 60-65 ml |

Donc, il n'y a pas de pénalisation en croissance et une faible pénalisation en fermentation pour le mutant. Il s'agit d'un mutant possédant le nouveau phénotype fil, objet de l'invention.

Les niveaux d'AMPc et de tréhalose ont été étudiés dans les deux souches portant la mutation *fil1* (PVD1050 et PVD1150) par rapport à leurs témoins respectifs (M5 *hxk2Δ* et M5). Le niveau d'AMPc a été mesuré sur les souches PVD1150 et M5, selon la méthode décrite par Thévelein et coll., 1987, J. Gen. Microbiol., 133, pp.2197-2205. Ce niveau d'AMPc a ainsi été déterminé après induction de sa synthèse par ajout de glucose (à 100 mM) sur des cellules ayant atteint la phase stationnaire après une croissance sur glycérol. Un signal AMPc atténué a été mis en évidence dans le mutant PVD1150 (figure 1-2). Le tréhalose a par ailleurs été dosé sur les souches PVD1150, PVD1050, M5 et M5 *hxk2Δ*, selon les conditions décrites par Neves et coll., 1991, FEBS Lett., 283, pp.19-22. Une mobilisation beaucoup moins rapide du tréhalose (après induction par le glucose sur des cellules en phase stationnaire) est observée dans les souches portant la mutation *fil1* que dans les souches témoins (figure 1-3).

A la vue des résultats des mesures de tréhalose et d'AMPc, il semble clair que la mutation *fil1* touche la voie Ras-AMPc. Toutefois, et contrairement aux mutants de la voie Ras-AMPc précédemment isolés et qui présentent des retards de croissance et de fermentation, le mutant *fil1* n'est que très faiblement pénalisé en croissance et en fermentation.

Afin de déterminer l'impact réel de la plus grande teneur en tréhalose sur la résistance au stress des mutants *fil1,* le gène *TPS1* (qui code pour la synthèse de tréhalose phosphate à partir de glucose) a été délété dans les souches *fil1* et les souches témoins de départ. Bien que ces souches *fil1 tps1Δ* ne synthétisent plus de tréhalose, elles conservent une bien meilleure résistance au stress thermique que les souches témoins *tps1Δ* : 30% de survie après 30 minutes à 52˚C contre 0,1% pour les témoins (conditions du test T1). Cela corrobore les résultats publiés par Van Dijck et coll. (1995, Appl. Environ. Microbiol,. 61, pp.109-115) qui mettaient en évidence que le tréhalose n'est pas, à lui seul, responsable de l'amélioration de la résistance au stress.

**B. Obtention de souches *fil300***

**a/ Obtention de la souche FD51 = HL816 *fil300***

Une mutagenèse a été réalisée sur la souche HL8.16 *leu2* (souche aneuploïde, ségrégeant de souche industrielle rendu auxotrophe pour la leucine ; collection LESAFFRE) : les cellules ont été cultivées à 30˚C dans un milieu YPD jusqu'à obtention de la phase stationnaire. Puis les cellules ont été incubées à 30˚C pendant 1 heure, en présence d'E.M.S. (Ethyl Methyl Sulfonate) selon la technique décrite dans " Methods in yeast Genetics, Cold Spring Harbor Laboratory Press" (Sherman et al., 1986) de manière à obtenir un taux de survie d'environ 10%. Après ce traitement, les cellules ont été lavées, remises en suspension dans du milieu YPD et cultivées à 30˚C jusqu'à l'obtention de la phase stationnaire. Du glucose a alors été ajouté dans la culture en phase stationnaire de manière à obtenir une concentration finale en glucose égale à 100 mM. Le milieu inoculé a été incubé à 30˚C pendant 30 minutes, puis à 56˚C pendant 30 minutes, et enfin à 65˚C pendant 30 minutes. Les cellules survivantes ont été isolées par étalement sur milieu YPD-A. Puis elles ont été soumises individuellement à un test de résistance à la chaleur, selon les conditions du test T1 avec une incubation de 30 minutes à 52˚C. A l'issue de cette première sélection, les six souches présentant les taux de survie les plus importants ont été retenues et utilisées pour un second traitement mutagène, cette fois aux ultra-violets (U.V.), selon la technique décrite par Spencer J.F.T. et Spencer D.M., Chapitre " Yeast genetics " extrait de " Yeast a practical approach ", 1988, Campbell et Duffus Eds. Dans le cadre de ce traitement, les cellules de chacune des 6 souches ont été cultivées dans un milieu YPD jusqu'à l'obtention de la phase stationnaire. Elles ont ensuite été lavées à l'eau, diluées puis étalées sur milieu YPD-A. Une dose de 30 mJ de lumière U.V. (de longueur d'onde égale à 260 nm) a été appliquée sur les boîtes ouvertes. Les 248 colonies ayant survécu au traitement mutagène ont été repiquées sur un milieu YPD-A puis incubées 2 heures à 60°C. A l'issue de ce traitement thermique, seules 2 colonies ont survécu. Un test de résistance à la chaleur en milieu liquide a alors été réalisé sur ces deux colonies, selon les conditions du test T1 avec une incubation de 30 minutes à 52°C après une incubation en présence de glucose pendant 1 heure à 30°C. La meilleure des 2 souches a été retenue pour un dernier traitement mutagène aux ultra-violets (U.V.), selon les conditions décrites précédemment mais avec une dose de 10 mJ de lumière U.V. (260 nm). Les colonies ayant survécu au traitement mutagène ont été repiquées sur un milieu YPD-A et incubées 6 heures à 60°C. A l'issue de ce traitement thermique, 171 colonies (sur près de 1500) ont survécu. Un test de résistance à la chaleur de ces 171 souches selon les conditions du test T1 a permis de sélectionner la souche FD51, nommée également HL816 *fil300*. Bien entendu, il a été vérifié que cette mutation était stable et qu'elle répondait à toutes les conditions du phénotype fil.

### b/ Etude de la souche HL816 *fil300*

Une très nette amélioration de la résistance à la chaleur de la souche a été confirmée par le test T1, que ce soit en phase stationnaire ou en reprise de fermentation (figure 1-4) . Par ailleurs, la mutation *fil300* induit une résistance à d'autres stress : en effet la souche HL816 *fil300* présente, dans les conditions du test T2, un taux de survie de plus de 50% après congélation de 12 jours à -20°C de cellules préfermentées 90 minutes à 30°C alors que le taux de survie du témoin est dans les mêmes conditions inférieur à 11% (figure 1-5). La souche HL816 *fil300* résiste donc également beaucoup mieux à la congélation que la souche témoin, que les cellules soient en phase stationnaire ou en reprise de fermentation.

Afin de mieux caractériser le phénotype lié à la mutation *fil300,* les niveaux de tréhalose et d'AMPc ont été mesurés sur des cellules en phase stationnaire qui sont soumises à une induction par le glucose. La souche témoin est la souche de départ HL8.16 *leu2.* En phase stationnaire (soit 0 minute de fermentation dans la figure 1-6), le niveau de tréhalose est 3 à 4 fois plus élevé dans le mutant *fil300* que dans le témoin HL8.16 *leu2.* En phase exponentielle, la vitesse de dégradation du tréhalose dans la souche HL8.16 *fil300* est globalement ralentie, par rapport à celle du témoin (figure 1-6). Il est également observé dans le mutant HL816 *fil300* une réponse AMPc réduite de près de 50% après une induction de la synthèse par le glucose sur des cellules en phase stationnaire (figure 1.7). Les performances de croissance et de force fermentative des souches HL8.16 *leu2* (témoin) et HL816 *fil300* ont été déterminées selon les tests T5 et A20. Les résultats sont donnés dans le tableau 1-C. La souche *fil300* conserve dans ces tests 80% des caractéristiques mesurées pour la souche de départ.

**Tableau 1-C**

|  | T5 Récolte (en matière sèche levure) 20 h [pour 8 g de mélasse] | A20 Force fermentative (ml $CO_2$ dégagé en 2 heures) |
|---|---|---|
| HL8.16 leu2 | 1.5 g | 85 |
| HL816 fil300 | 1.2 g | 68 |

### b/ Isolement de la souche HL822 *fil300*

Les pertes de rendement de croissance et de force fermentative du mutant HL816 *fil300* qui sont au maximum de ce qui est toléré dans le cadre du phénotype fil pénalisent son utilisation. C'est pourquoi il a été réalisé des croisements entre ledit mutant et quelques ségrégeants afin d'isoler, dans la descendance des diploïdes obtenus des ségrégeants thermorésistants aux performances améliorées. La souche HL822 *fil300*, également nommée FDH16-22, a ainsi été isolée. Cette souche, qui est un ségrégeant issu d'un croisement entre la souche HL816 *fil300* et le ségrégeant HL816 (collection LESAFFRE), possède un rendement de croissance sur mélasse et une force fermentative identiques à ceux de la souche témoin HL8.16 *leu2* (tableau 1-D). Elle possède par ailleurs un niveau de thermorésistance identique à celui de la souche HL816 *fil300*.

**Tableau 1-D** : Rendement de croissance sur mélasse et force fermentative (mesurée dans le test A20) des souches HL8.16 *leu2* et HL.822 *fil300*.

| | T5<br>Récolte (en matière sèche levure)<br>après 20 h [pour 8 g de mélasse] | A20<br>Force fermentative<br>(ml $CO_2$ dégagé en 2 heures) |
|---|---|---|
| HL8.16 leu2 | 1.5 g | 85 |
| HL822 fil300 | 1.5 g | 85 |

Pour chacune des deux souches *fil300* (HL816 *fil300* et HL822 *fil300*), la stabilité en pâtons congelés a été comparée à celle de la souche témoin HL8.12 *leu2* par mesure de l'évolution de l'activité fermentative en fonction de la durée de congélation selon les conditions du test C2.

Une très nette amélioration de la stabilité en congélation a été observée pour les souches *fil300* par rapport au témoin HL8.16 *leu2* (tableau 1-E). Ainsi, les souches *fil300* conservent au moins 60% de leur force fermentative après 1 mois de conservation à la température de -20˚C alors que le témoin ne conserve que 40% de sa force fermentative dans les mêmes conditions (tableau 1-E).

**Tableau 1-E** : Stabilité en congélation, mesurée par le ratio entre la force fermentative au Nième jour de mesure et la force fermentative au premier jour de congélation (Test C2).

| | Test C2<br>Stabilité en congélation (%) | | |
|---|---|---|---|
| | 1 jour | 28 jours | 42 jours |
| HL8.16 leu2 | 100 % | 41% | 39% |
| HL816 fil300 | 100 % | 60 % | 53% |
| HL822 fil300 | 100 % | 62 % | 57% |

## EXEMPLE 2 : Utilisation de cycles de congélation/décongélation comme outil d'isolement de mutants fil à partir d'une souche industrielle

La souche de départ est la souche S47 déposée au C.N.C.M., 25 rue de Docteur Roux, 75724 Paris, France, sous le numéro I-2037. C'est une souche de levure de panification aneuploïde utilisée industriellement, c'est probablement la meilleure souche utilisée actuellement en France pour la panification en cru surgelé. Elle a été cultivée dans un milieu liquide YPD à 30˚C jusqu'à l'obtention de la phase stationnaire. Un traitement mutagène aux U.V. a alors été réalisé selon la technique décrite par Spencer J.F.T. et Spencer D.M., Chapitre " Yeast genetics" extrait de " Yeast a practical approach " (1988), Campbell et Duffus Eds., de manière à obtenir un taux de survie d'environ 10%. Les cellules ont ainsi été traitées avec une dose d'U.V. (260 nm) de 5 mJ. Elles ont ensuite été cultivées à 30˚C sur milieu YE-mélasse gélosé (agar 20 g/l, extrait de levure 5 g/l, mélasse 5 g/l, $(NH_4)_2HPO_4$ 0.5 g/l, pH 5.0-5.5), puis ont été récoltées après 2 à 3 jours par lavage des boites avec de l'eau. Des mini-pâtons de 0,5 g ont été façonnés à partir des levures récoltées, selon la composition suivante : farine 56.5 %,

NaCl 1.0 %, eau 42.5 %, levures $4.10^8$ cellules/gramme de pâton. Ces mini-pâtons ont été incubés à 30˚C pendant 30 minutes. Puis ils ont été soumis à 200 cycles successifs de congélation à -30˚C et décongélation à température ambiante, de manière à n'obtenir que quelques milliers de cellules survivantes (taux de survie inférieur à 0.01%), qui ont été isolées des pâtons.

La résistance à la congélation a ensuite été testée individuellement sur les cellules survivantes, par mesure du taux de survie des cellules après une congélation et par mesure de la perte de capacité fermentative après congélation. Parallèlement, le rendement de croissance sur mélasse et la force fermentative des meilleures souches ont été mesurés.

* Le taux de survie après congélation est mesuré selon les conditions du test T2 avec une phase fermentaire de 30 minutes à 30˚C, suivi d'une congélation à -30˚C pendant 24 heures, puis d'une décongélation à température ambiante.

* La croissance des souches a été mesurée par le rendement obtenu après une culture de 20 heures sur milieu mélasse gélosé selon le test T5 ; les levures récoltées sont utilisées pour mesurer la force fermentative à 30˚C et sur 2 heures, selon les conditions du test A1.

Sur l'ensemble des souches ayant survécu aux traitements successifs de congélation/décongélation en mini-pâtons, 7 souches nommées AT25, AT26, AT27, AT28, AT29, AT30 et AT31 ont été pré-sélectionnées. Les résultats des taux de survie après congélation (test T2) sont donnés dans le tableau 2-A, les rendements de croissance sur mélasse (T5) et la force fermentative (A1) sont donnés dans le tableau 2-B. Même si l'ensemble de ces mutants possèdent un taux de survie (tableau 2-A) bien meilleur que celui du témoin, il faut tenir compte des rendements de croissance et de la force fermentative de chaque mutant pour la sélection de souches répondant aux critères du phénotype fil (tableau 2-B). C'est pourquoi seules les souches AT25, AT26, AT28 et AT31 ont été retenues à l'issue de ces tests. Des mesures de taux de croissance maximal ont été réalisées selon le test T4 sur ces différents mutants et la souche témoin S47, les résultats sont présentés dans le tableau 2-C.

**Tableau 2-A**

| | Test T2 Taux de survie (%) après : | |
|---|---|---|
| | congélation (24 heures à -25˚C) sans fermentation préalable | congélation (24 heures à -25˚C) avec fermentation préalable 30 minutes à 30˚C |
| S47 | 36 | 17 |
| **AT25** | 51 | 47 |
| **AT26** | 59 | 42 |
| **AT27** | 45 | 40 |
| **AT28** | 47 | 39 |
| **AT29** | 72 | 32 |
| **AT30** | 47 | 28 |
| **AT31** | 38 | 27 |

**Tableau 2-B :** Rendement de croissance (exprimé par le ratio entre la quantité de levure produite en 20 heures exprimée à 30% de matières sèches par rapport à la quantité de mélasse utilisée ramenée à 50% de sucre) et force fermentative (exprimée par la quantité de $CO_2$ dégagée en 2 heures à 30˚C) des mutants de la souche S47 et de la souche S47 de départ.

| | Test T5 Rendement de croissance (%) | Test A1 Force fermentative (ml CO2) |
|---|---|---|
| S47 | 67 % | 138 |
| **AT25** | 64 % | 116 |
| **AT26** | 62 % | 116 |
| **AT27** | 47 % | 113 |
| **AT28** | 62 % | 120 |
| **AT29** | 51% | 64 |
| **AT30** | 62 % | 84 |
| **AT31** | 62 % | 133 |

**Tableau 2-C :** Taux de croissance maximal de la souche S47 et des mutants isolés à partir de la souche S47, sur différents milieux de croissance selon le test T4.

| SOUCHE | TAUX DE CROISSANCE MAXIMAL ou $\mu$max (h$^{-1}$) | | |
|---|---|---|---|
| | glucose 100 mM | glucose 10 mM | mélasse 0,5% |
| S47 | 0,69 | 0,60 | 0,57 |
| AT25 | 0,60 | 0,61 | 0,54 |
| AT26 | 0,67 | 0,56 | 0,56 |
| AT27 | 0,37 | 0,23 | 0,26 |
| AT28 | 0,61 | 0,62 | 0,60 |

(suite)

| SOUCHE | TAUX DE CROISSANCE MAXIMAL ou $\mu$max (h$^{-1}$) | | |
| --- | --- | --- | --- |
| | glucose 100 mM | glucose 10 mM | mélasse 0,5% |
| AT29 | 0,59 | 0,48 | 0,52 |
| AT30 | 0,62 | 0,55 | 0,56 |
| AT31 | 0,63 | 0,60 | 0,57 |

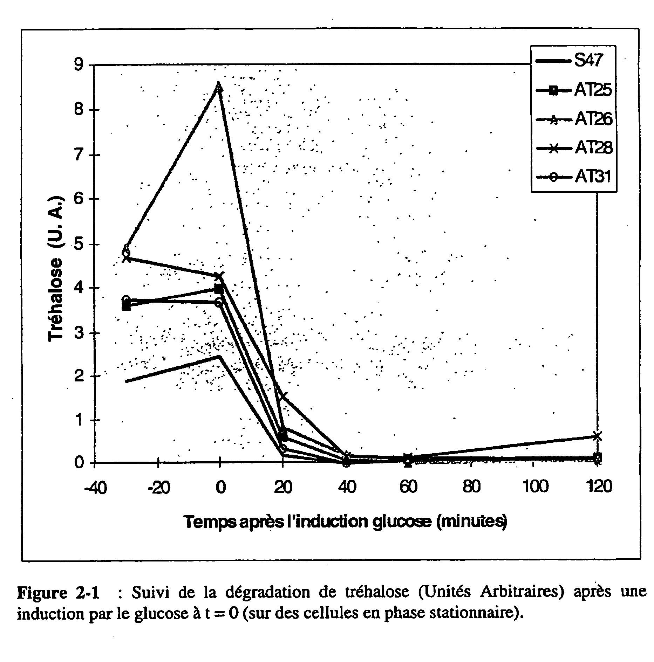

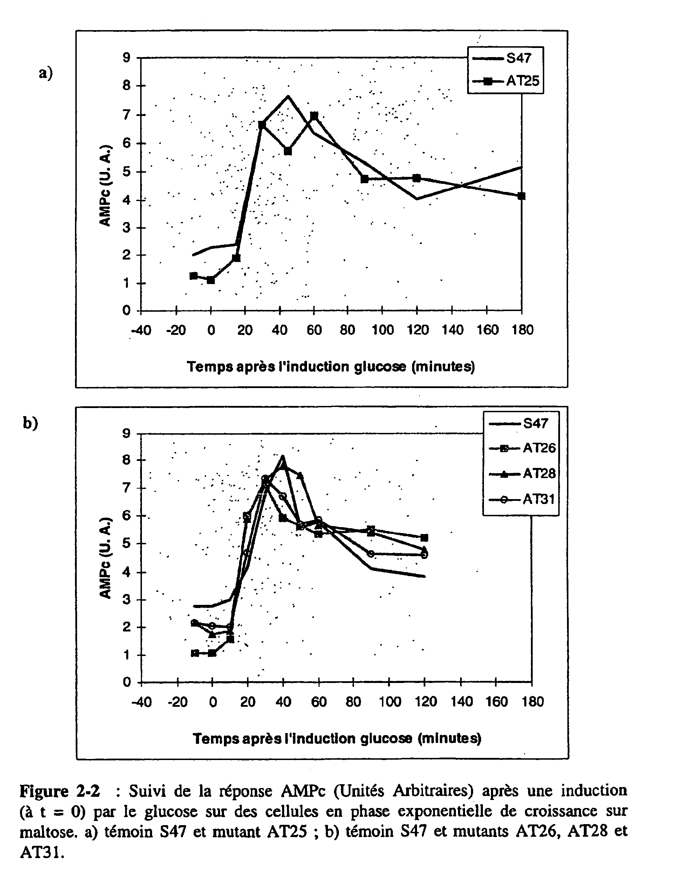

Afin de voir si les mutations portées par les souches AT25, AT26, AT28 et AT31 affectaient également la voie Ras-AMPc comme dans le cas des souches fil décrites dans l'exemple 1, les niveaux d'AMPc et de tréhalose dans ces différentes souches ont été mesurés. Les mesures de tréhalose ont été réalisées sur des cellules cultivées jusqu'à la phase stationnaire puis soumises à une induction de sa mobilisation ou dégradation par ajout de glucose (100 mM). Le niveau initial de tréhalose, qui correspond au niveau de tréhalose en phase stationnaire, est plus élevé pour tous les mutants que pour le témoin (t=0, figure 2-1). En présence de glucose, la dégradation du tréhalose est assez rapide pour l'ensemble des souches (mutants et témoin) puisque après 20 minutes et plus, un niveau faible et presque identique de tréhalose est obtenu pour l'ensemble des souches. Or le stress imposé dans le cadre du test T2 est réalisé après une incubation de 30 minutes en présence de glucose. Cela confirme, là encore, que le tréhalose ne permet pas d'expliquer les meilleures performances de ces mutants fil. En ce qui concerne l'AMPc, les mesures ont été réalisées sur des cellules en phase exponentielle de croissance sur maltose qui ont été soumises à une induction par du glucose (100 mM). Dans ces expériences, il n'y a pas de réduction significative du signal AMPc dans les mutants par rapport à la souche témoin S47 (figures 2-2, a & b). Il semblerait donc que les mutants cryorésistants AT25, AT26, AT28 et AT31, qui ont été isolés suite à des cycles successifs de congélation/ décongélation et qui ont le phénotype fil, portent des mutations qui touchent d'autres cibles que la voie Ras-AMPc.

**EXEMPLE 3 : Identification de la mutation *fil1* et reconstruction de souches portant la mutation *fil1***

Des croisements entre la souche PVD1150 et d'autres souches ont montré que la mutation *fil1* était localisée à proximité d'un centromère. La souche PVD1150 a donc été recroisée avec différentes souches haploïdes portant des marqueurs génétiques situés à proximité des centromères de chaque chromosome et une analyse a été effectuée sur les tétrades issues de la sporulation des diploïdes obtenus.

Il a ainsi été déterminé que la mutation *fil1* était localisée à proximité du centromère du chromosome X. La souche PVD1150 a donc été complémentée avec chacun des gènes situés dans cette région, selon la stratégie générale suivante :

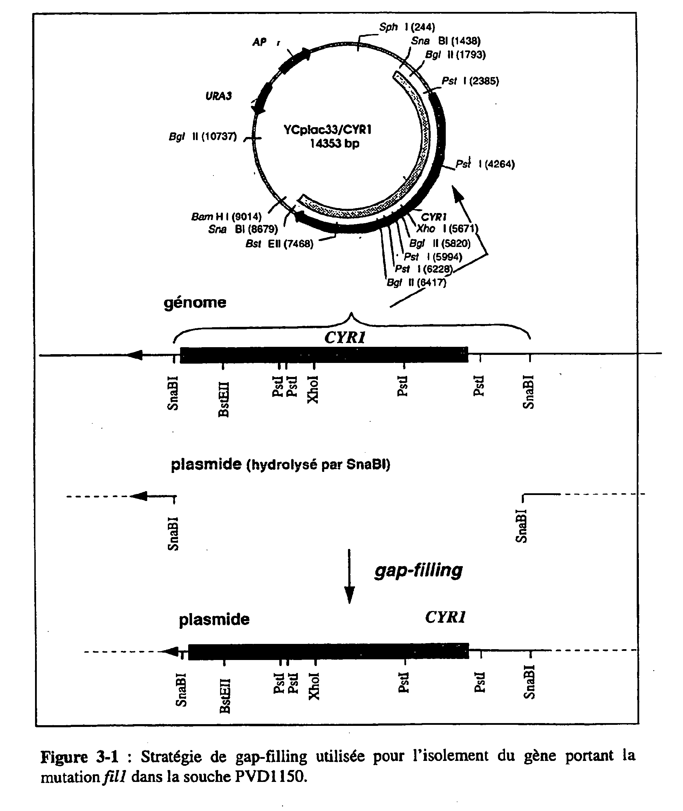

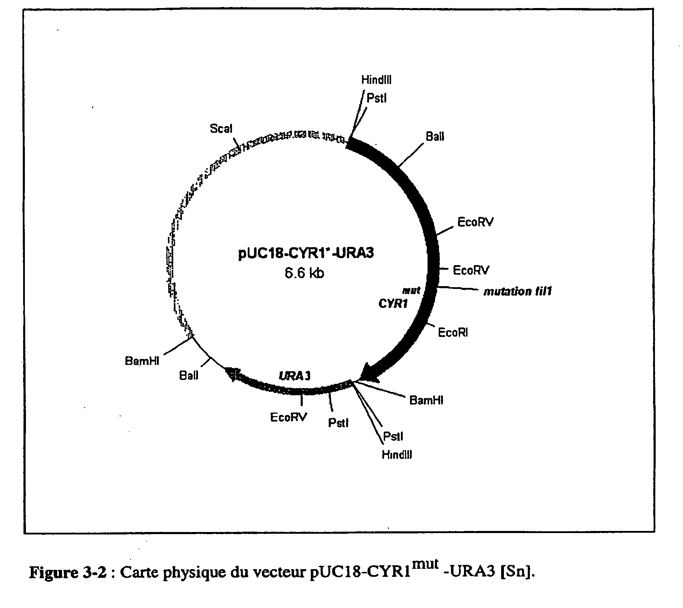

- transformation de la souche PVD1150 par des plasmides centromériques portant chacun un des gènes situés à moins de 25 kb du centromère du chromosome X,
- recherche des clones transformants ayant perdu le phénotype de thermorésistance,
- isolement du gène portant la mutation à partir du mutant PVD1150 par la technique de " gap-filling " définie ci-après et illustrée par la figure 3-1 ; retransformation de la souche PVD1150 avec le gène muté ainsi isolé de manière à vérifier qu'il ne s'agit pas d'un gène suppresseur,
- séquençage du gène muté issu de PVD1150 et de son allèle sauvage issu de M5 pour identifier et localiser la mutation,
- réintroduction par recombinaison homologue du gène muté dans au moins une autre souche de laboratoire thermosensible et vérification de l'obtention du phénotype fil démontrant la monogénicité de la mutation et sa non dépendance du contexte génétique de la souche mutée d'origine PVD1150.

Cette stratégie de complémentation a été réalisée de la manière suivante : toutes les constructions ont été réalisées selon les techniques habituelles et notamment selon l'ouvrage " MOLECULAR CLONING", J. Sambrook, E.F. Fritsch, T. Maniatis, Cold Spring Harbor Laboratory Press, 1989.

1/ Transformation de la souche PVD1150 par des plasmides centromériques portant chacun un des gènes situés à moins de 25 kb du centromère du chromosome X

Chaque gène situé à moins de 25 kb du centromère a été amplifié par PCR, puis cloné dans le vecteur YCplac33 (Gietz R.D. et Sugino A. (1988), Gene, 74, pp.527-534) qui porte le gène *URA3.* Chacun des vecteurs obtenus est utilisé pour transformer la souche PVD1150 (auxotrophe pour l'uracile). La présence des plasmides est ensuite vérifiée sur les transformants obtenus.

## 2/ Recherche des clones transformants ayant perdu le phénotype de thermorésistance

Les différents transformants portant chacun, sur un vecteur monocopie, l'un des gènes situés au plus à 25 kb du centromère du chromosome X, ont été testés pour leur perte de thermorésistance. Chaque souche PVD1150 portant l'un des gènes situés autour du centromère du chromosome X est déposée en stries sur un milieu YPD-A préchauffé à 57°C, qui est incubé pendant 90 minutes à 57°C. En parallèle, la souche PVD1150 qui porte un plasmide monocopie sans insert, en l'occurrence le vecteur YCplac33 est utilisée comme témoin non complémenté. Après ce traitement thermique, les souches sont incubées à 30°C jusqu'au développement de la croissance du témoin (apparition d'un tapis cellulaire). Les souches portant un gène qui complémente la mutation *fil1* sont celles qui ne se sont pas encore développées.

Les résultats de ces tests ont permis de montrer que la mutation *fil1* était localisée dans le gène *CYR1* (nommé également CDC35), puisque seul ce gène était capable de complémenter le phénotype fil dans la souche PVD1150. Le gène *CYR1/CDC35* code pour l'adénylate cyclase, enzyme de la voie Ras-AMPc qui permet la synthèse d'AMPc à partir d'ATP . Ce résultat est cohérent avec le fait que la souche PVD1150 présente un niveau réduit d'AMPc (exemple 1).

De manière à vérifier que la perte de thermorésistance était effectivement liée à la présence du gène *CYR1/CDC35* sauvage apporté par le plasmide, la souche PVD1150 contenant le vecteur YCplac33-CYR1 a été cultivée sur du milieu YPD, un milieu non sélectif permettant de perdre rapidement le plasmide. Des colonies ont été isolées de cette culture sur un milieu YPD-A, puis répliquées sur milieu sélectif SD-URA (milieu dépourvu d'uracile) pour rechercher les clones qui ne poussent pas et qui ont donc perdu le plasmide. Un test de thermorésistance identique au précédent confirme que l'ensemble de ces clones ayant perdu le plasmide sont redevenus thermorésistants.

## 3/ Isolement du gène portant la mutation à partir du mutant PVD1150

La délétion du gène *CDC35/CYR1* est connue pour entraîner la létalité des souches. Il était indispensable d'isoler le gène muté.

Le gène muté de la souche PVD1150 a été cloné par la technique de " gap-filling " [Iwasaki, T. et coll. (1991) Gene, 109, pp.81-87] ou " allele rescue" [Orr-Weaver, T.L. et coll. (1983) " Methods Enzymol. ", 101, pp.228-245].

La méthode a été appliquée à la souche PVD1150 en la transformant avec le plasmide YCplac33-CYR1 digéré par l'enzyme *Sna*BI (figure 3-1). Les transformants poussant sur milieu minimum dépourvu d'uracile (milieu SD-URA) ont ensuite été sélectionnés. Ces transformants ne peuvent croître que s'il y a eu recircularisation du vecteur, notamment par un événement de double recombinaison ayant intégré la partie manquante du gène grâce au gène *CYR1* muté dans la souche PVD1150 (figure 3-1). Parallèlement, la souche M5 a été transformée avec le vecteur YCplac33-CYR1 digéré par *Sna*BI, puis les transformants ont été sélectionnés sur milieu minimum, de manière à obtenir un vecteur portant le gène *CYR1* non muté issu de la souche témoin M5.

La souche PVD1150 a alors été retransformée :

- avec le vecteur YCplac33-CYR1mut qui porte le gène muté issu du vecteur YCplac33-CYR1 digéré par *Sna*BI et rempli par gap-filling dans la souche PVD1150,
- avec le vecteur YCplac33-CYR1/S qui porte le gène sauvage issu du vecteur YCplac33-CYR1 digéré par *Sna*BI et rempli par gap-filling dans la souche M5.

Le niveau de thermorésistance de ces deux souches a ensuite été comparé à l'aide du test suivant : dépôt des cellules en stries sur milieu gélosé YPD-A préchauffé à 57°C et incubé à 57°C pendant 90 minutes. Comme attendu, le gène muté ne complémente pas la mutation ; en revanche, le gène *CYR1* sauvage rend la souche PVD1150 thermosensible.

## 4/ Séquençage du gène muté (issu de PVD1150) et de son allèle sauvage (issu de M5) pour identifier et localiser la mutation

Le gène muté *CYR1mut* issu de PVD1150 et le gène *CYR1* sauvage issu de M5 ont été séquencés en parallèle par la technique de séquençage direct sur produits de PCR obtenus par amplification de fragments couvrant tout le gène, ce qui a permis la localisation de la mutation *fil1*. Il s'agit d'un changement d'une base G en une base A en position n°5044 de la partie codante du gène *CYR1* (gène référencé sous le nom YJL005w chez *Sacccharomyces cerevisiae*). Cela correspond également à un changement en position 429888 du chromosome X, selon la classification du MIPS (Munich Information Center for Protein Sequence). Ce changement est représenté dans le tableau ci-dessous.

|                | Souche témoin |        |                | Souche *fil1* |        |
|----------------|:-------------:|--------|----------------|:-------------:|--------|
|                | glu           |        |                | lys           |        |
| GG             | GAG           | CT     | GG             | AAG           | CT     |
| CC             | CTC           | GA     | CC             | TTC           | GA     |

La mutation a été vérifiée à la fois par le séquençage d'un mélange de trois produits PCR indépendants et aussi par le séquençage du brin d'ADN complémentaire. Ce changement conduit au passage d'un acide glutamique en lysine en position 1682 de la protéine. Il intervient dans la région codant pour le site catalytique de l'enzyme, et à proximité de la région qui est supposée être impliquée dans l'activation de l'adénylate cyclase par les protéines Ras :

| 0 | | | | | 2026 |
|---|---|---|---|---|---|
| Domaine N-terminal (régulation négative) | Région riche en Leucine (domaine de fixation membranaire) | Région interdomaines (réponse Ras) | Domaine catalytique | | Inter-action Cap |

657      1301      1667      1891

1682 (*fil1*)

5/ Réintroduction (par recombinaison homologue) du gène muté dans des souches de laboratoire thermosensibles et étude du phénotype associé

a/ Construction du plasmide pUC18-CYR1^mut- URA3 [Sn]

Le fragment PstI-BamHI du vecteur Ycplac33-CYR1^mut, fragment qui contient la partie 3' du gène *CYR1*^mut (mutation *fil1* incluse) a été sous-cloné dans le vecteur pUC18. Puis le marqueur d'auxotrophie *URA3,* issu du vecteur pJJ242 (Jones et Prakash (1990) Yeast, 6, pp.363-366) a été inséré dans ce nouveau vecteur, en aval de la partie codante du gène *CYR1*^mutdans la région 3' non codante, au niveau d'un site de restriction unique (*Sna*BI). Le vecteur obtenu, nommé pUC18-CYR1^mut-URA3 est présenté dans la figure 3-3.

b/ Transformation des souches haploïdes de levures M5 et SP1

Le vecteur pUC18-CYR1^mut -URA3 a été hydrolysé par l'enzyme appropriée (en l'occurence *Bal*l), ce qui a permis de libérer un fragment linéaire de 3,2 kb contenant la *mutation fil1,* le marqueur *URA3* ainsi que des séquences aux extrémités permettant la recombinaison homologue. Ce fragment a été utilisé pour transformer des souches de laboratoire : la souche M5 (ura3, trp1, leu2) qui est un haploïde vrai 16n issu de la souche M5 2n décrite par Schaaf et coll. (1989) Curr. Genet., 15, pp.78-81, et la souche SP1 (ura3, trp1, leu2, ade8) décrite par Toda et coll. (1985) Cell, 40, pp.27-36. La présence de la mutation a ensuite été recherchée sur quelques uns des transformants ainsi obtenus, par séquençage de la zone portant en principe la mutation. Cela nous a permis d'isoler des souches M5 *fil1* et SP1 *fil1*.

c/ Recherche du phénotype de thermorésistance

La thermorésistance des souches M5 *fil1* et SP1 *fil1* a été comparée à celle de leurs témoins respectifs (M5 et SP1) selon les conditions du test T1 avec une incubation en présence dé glucose de 30 minutes à 30˚C et un traitement thermique de 20 minutes à 52˚C.
Les résultats concernant les souches M5 *fil1* et SP1 *fil1* reconstruites, qui sont présentés dans le tableau 3-A montrent que ces souches présentent effectivement un phénotype de thermorésistance, qui n'est pas observé dans les souches témoin.

**Tableau 3-A :** Viabilité résiduelle après choc thermique à 52˚C des souches *fil1* reconstruites et de leurs témoins.

| Souche d'origine | Viabilité résiduelle après traitement à 52˚C (%) | |
| --- | --- | --- |
| | souche sauvage | souche *fil1* |
| M5 | 9% | 96 % |
| SP1 | 1% | 16 % |

d/ Remarque

La mutation *fil1* est une mutation ponctuelle sur le gène *CDC35/CYR1.* Une délétion de ce gène est létale, les mutants cyr1 décrits jusqu'alors conduisaient à des taux de croissance très réduits.

Dans la souche mutée *fil1,* le gène muté *CDC35/CYR1* est surexprimé, semble-t-il pour compenser l'effet de la mutation, étant entendu que le niveau d'AMP cyclique reste plus faible. La réponse à la mutation *fil1* semble correspondre à un équilibre métabolique complexe, qui peut dépendre du contexte (= background) génétique de la souche.

Par ailleurs, il faut signaler que la mutation *fil1* semble retarder de manière importante la germination des spores contenant cette mutation. Cette déficience au niveau de la méiose ne se retrouve pas en phase active. Il faut seulement en tenir compte au niveau des constructions de souches *fil1* par génétique classique.

## EXEMPLE 4 : Caractérisation de la mutation *fil2*

La souche KL1(=W303 *fil2*) a été isolée à partir de la souche W303-1A (Thomas et Rothstein (1989) Cell, 56, pp.619-630) selon un protocole de recherche de souches fil similaire à ceux développés dans l'exemple 1. La mutation *fil2* est monogénique et récessive. Dans un premier temps, un test de thermorésistance a permis de confirmer l'amélioration apportée par la mutation *fil2*. Il a ensuite été mis en évidence une résistance croisée aux stress. Il a été constaté que la souche mutée *fil2* était bien une mutation fil, car cette souche mutée n'était pas affectée significativement par rapport à la souche de départ quant à sa croissance ou son activité fermentative. Comme il s'agit d'une souche de laboratoire, le gène portant la mutation a été recherché.

## a/ Résistances aux stress

Pour mesurer la résistance aux stress thermiques (chaleur et congélation), les souches KL1 et W303-1A ont été cultivées sur milieu YPD à 30˚C jusqu'à l'obtention de la phase stationnaire. Après centrifugation, les cellules ont été resuspendues dans du milieu YP et incubées à 30˚C. Au bout de 30 minutes, du glucose a été ajouté jusqu'à atteindre une concentration finale de 100 mM dans le milieu. Dans le cas du stress à la chaleur, l'incubation à 30˚C a été poursuivie pendant 30 minutes, puis les cellules ont été incubées à 52˚C pendant 30 minutes. Dans le cas de la congélation, l'incubation à 30˚C a été poursuivie pendant 90 minutes, puis les cellules ont été resuspendues dans du milieu YP glacé et congelées à -30˚C pendant 2 jours puis décongelées à 30˚C. Quatre cycles successifs de congélation/ décongélation ont été effectués.

Ces deux tests ont permis de montrer qu'il existait une corrélation entre la thermorésistance et la résistance à la congélation dans le cas de la mutation *fil2*. La souche KL1 présente ainsi un taux de survie de 80% après traitement à la chaleur contre 20% pour la souche témoin W303-1A ; de même après congélation dans le test sévère effectué, le taux de survie du mutant KL1 est près de 4 fois supérieur à celui du témoin : environ 30% pour KL1 contre environ 8% pour W303-1A.

## b/ Localisation du gène muté

Une stratégie identique à celle ayant permis la localisation de la mutation *fil1* (cf. exemple 3) a été réalisée. Il a ainsi été déterminé que la mutation *fil2* était localisée à proximité du centromère du chromosome IV. La souche KL1 a donc été complémentée avec chacun des gènes situés dans cette région, selon la stratégie générale décrite dans l'exemple 3 :

- transformation de la souche KL1 (auxotrophe pour l'uracile) par des plasmides centromériques portant chacun un des gènes situés à moins de 60 kb du centromère du chromosome X
- recherche des clones transformants ayant perdu le phénotype de thermorésistance

- isolement du gène portant la mutation à partir du mutant KL1 ; retransformation de la souche KL1 avec le gène ainsi isolé de manière à vérifier qu'il ne s'agit pas d'un gène suppresseur.

Les résultats des tests de complémentation ont permis de montrer que la mutation *fil2* était localisée dans le gène *GPR1*. Ce gène code pour un récepteur potentiel couplé à une protéine G et il est supposé initier la voie de signalisation associée à la protéine G codée par le gène *GPA2* (Xue, Y et coll. (1998) EMBO Journal 17 : 1996-2007).
De manière à vérifier que le phénotype de thermorésistance était effectivement associé à une mutation sur le gène *GPR1,* le niveau de thermorésistance de la souche KL1 a été comparé à celui de la souche W303-1A dans laquelle le gène *GPR1* a été délété (délétion non létale), ainsi qu'à celui du témoin thermosensible W303-1A. Les résultats montrent clairement que la délétion du gène *GPR1* permet d'obtenir un niveau élevé de thermorésistance. Par ailleurs, le diploïde obtenu par croisement entre KL1 et W303-1A *gpr1*Δ présente un niveau de thermorésistance aussi élevé que celui des souches KL1 et W303-1A *gpr1*Δ, ce qui confirme que la mutation *fil2* touche effectivement le gène *GPR1*.
Le gène *GPR1* muté de manière à conférer le phénotype *fil2* peut être isolé selon la technique décrite à l'exemple 4 et il permettra de transformer des souches industrielles de manière à obtenir des souches industrielles ayant le phénotype *fil2*.
Dans l'allèle *fil2* du gène *GPR1,* la mutation point suivante a été trouvée : la base 948 à partir du départ de la phase codante, c'est-à-dire de l'ATG, est changée, une Thymine (T) devient une Adenine(A). Le codon dans la souche sauvage est TAT qui code pour une Tyrosine en position 316 dans la protéine, il est changé en TAA qui code un STOP. L'allèle *fil2* apparaît coder pour une version tronquée de la protéine Gpr1 qui contient en tout 962 acides aminés. L'acide aminé 316 avant lequel s'arrête la synthèse de la protéine *fil2* tronquée, est situé dans la 3ème boucle intracellulaire de la protéine réceptrice Gpr1. C'est cette boucle qui est connue pour interagir dans les protéines réceptrices de mammifères avec la protéine G, c'est probablement cette boucle qui interagit dans la levure avec la protéine Gpa2.

**c/ Réintroduction de la mutation dans une souche diploïde**

Une souche diploïde vraie, non auxotrophe, relativement proche des souches industrielles, a été transformée de manière à ce que ses deux gènes *GPR1* soient remplacés par le gène muté, correspondant à l'allèle *fil2*. La souche diploïde de départ et la souche diploïde *fil2* ont été cultivées sur mélasses en fed-batch, en fermenteurs pilote. Les levures obtenues ont fait l'objet d'un essai de séchage et de réalisation de pâtons congelés. On note une légère diminution de la perte de force au séchage, à la limite de la signification, avec la souche diploïde *fil2* par rapport à la souche de départ. On note une très nette amélioration de la stabilité des pâtons congelés qui après 15 jours à -20˚C est multipliée par environ 1,5.

**EXEMPLE 5 : Performances de souches fil en séchage**

Le phénotype fil est intéressant pour obtenir des levures sèches plus actives, car il permet de soumettre au séchage des biomasses plus actives, sans perte dramatique d'activité fermentative lors du séchage.
Les deux souches *fil1* reconstruites obtenues comme décrit dans l'exemple 3 et la souche S47 *fil500* (= AT28) obtenues comme décrit dans l'exemple 2 et les souches de départ correspondantes ont été cultivées selon des procédés classiques en fed batch en cuve pilote de quelques litres utiles comme décrit dans les brevets US 4318929, 4318930 et 4396632 de manière à obtenir une biomasse active à plus de 7% d'azote sur matières sèches. Cette biomasse est ensuite séchée par un procédé type fluidisation comme décrit dans les brevets US cités ci-dessus.

**a/ Séchage des 2 souches *fil1* reconstruites et des 2 souches de départ M5 et SP1**

La force fermentative de la biomasse obtenue avec chacune des 4 souches avant séchage, c'est-à-dire sous forme fraîche à environ 30% de matières sèches a été mesurée selon le test A20. La force fermentative des levures sèches à environ 95% de matières sèches a été mesurée selon le test A'20. Comme dans le cadre d'un tel test pilote, les forces fermentatives en valeur absolue n'ont guère de signification, on a affecté la perte au séchage obtenue avec chaque souche de départ du coefficient 100 et on a exprimé la perte au séchage des souches reconstruites *fil1* obtenues selon l'exemple 3 en pourcentage de la perte au séchage de la souche de départ. Ces résultats sont donnés dans le tableau 5A.

**Tableau 5-A :** Evaluation de la perte de force au séchage des souches *fil1* reconstruites et de leur témoin.

| | Perte de force des levures sèches exprimée en pourcentage de la perte de force du témoin | |
| --- | --- | --- |
| | réhydratation à 20˚C | réhydratation à 38˚C |
| M5 | 100 | 100 |
| M5 *fil1* | 44 | 26 |
| SP1 | 100 | 100 |
| SP1 *fil1* | 49 | 52 |

Ces résultats montrent que le gène *fil1*, c'est-à-dire le gène *CYR1/CDC35* portant la mutation *fil1* permet de construire des souches industrielles qui présenteront une perte au séchage inférieure à celle des souches connues jusqu'à maintenent.

**b/ Séchage de la souche S47 et de la souche S47 *fil500***

La même expérience a été réalisée avec la souche S47 *fil500* obtenue dans le cadre d'une sélection basée sur la résistance à la congélation en phase de fermentation, compte tenu que très souvent les résistances au stress sont croisées. Les forces fermentatives ont été mesurées selon le test A1 pour la levure fraîche, et le test A'1 pour la levure sèche avec réhydratation à 20˚C. Les résultats sont donnés dans le tableau 5-B.

**Tableau 5-B :** Evaluation de la perte de force au séchage de la souche S47 *fil500* et de son témoin.

| | Perte de force des levures sèches exprimée en pourcentage de la perte de force du témoin |
| --- | --- |
| | réhydratation à 20˚C |
| S47 | 100 |
| S47 fil500 | 66 |

Ce tableau 5-B confirme que le phénotype fil permet de diminuer la perte au séchage des levures très actives et donc d'obtenir des levures sèches très actives.

**c/ Séchage de la souche AT25 = *S47 fil400* et de la souche S47**

L'expérience ci-dessus a été répétée plusieurs fois avec la souche AT25 = S47 *fil400* qui, dans le cadre de l'ensemble des essais, est la meilleure souche de la série des souches AT construites dans le cadre de l'exemple 2.
Les résultats moyens suivants ont été obtenus :

| | N/MS azote sur matière sèche | Test A1 sur levure avant séchage | Test A'1 sur levure sèche à 95 % matières sèches | Perte après séchage et réhydratation à 38˚C |
| --- | --- | --- | --- | --- |
| | | ml $CO_2$ | ml $CO_2$ | |
| S47 | 8,2% | 152 | 105 | 31% |
| AT25 | 8,3% | 165 | 135 | 18% |

**EXEMPLE 6 : Utilisation de souches fil en pâtons congelés**

Dans cet exemple, sont décrits les résultats en congélation de souches fil obtenues dans l'exemple 2 : la souche S47 *fil400* (=AT25), la souche S47 *fil500* (=AT28) et la souche AT26. La stabilité en congélation de ces souches fil est comparée à celle de la souche S47, souche témoin dont elles sont issues. Pour cela, il a été suivi la perte de dégagement de $CO_2$ associée à une conservation prolongée dans des pâtons congelés à -20˚C.
Les levures sont cultivées sur mélasse, en fed batch sur fermenteur pilote de manière à obtenir des biomasses actives ayant une teneur en azote maximale de 8% sur matières sèches, puis utilisées pour façonner des pâtons, selon les conditions décrites dans le test C1. Pour chaque souche, la valeur de dégagement gazeux de référence correspond à la valeur de dégagement gazeux mesurée après 1 jour de conservation à -20˚C. Les autres pâtons sont décongelés après 1 semaine, 2 semaines, 3 semaines, 4 semaines et 6 semaines ; ils permettent de suivre l'évolution de activité fermentative en fonction de la durée de conservation à -20˚C. Par ailleurs, la stabilité en pâton de chaque souche après n jours de conservation à -20˚C est mesurée par le rapport suivant :

$$\frac{\text{dégagement de } CO_2 \text{ selon test C1 au nième jour de conservation à -20°C}}{\text{dégagement de } CO_2 \text{ selon test C1 après le premier jour de conservation à -20°C.}}$$

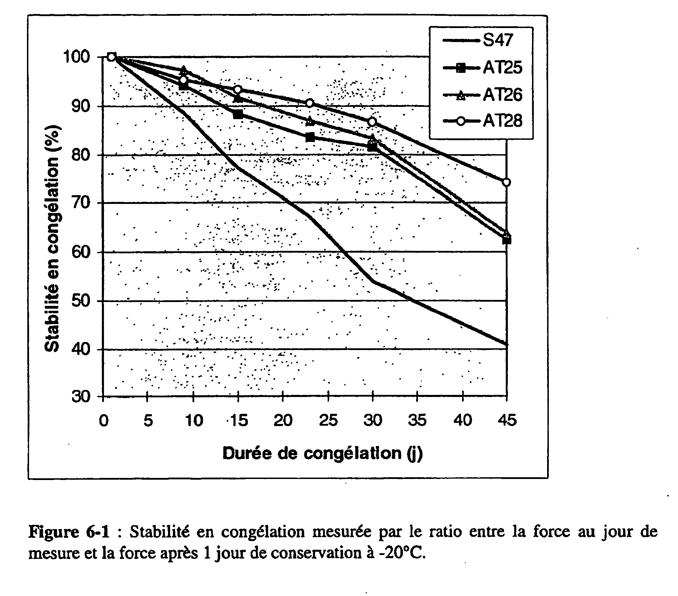

Les résultats sont présentés dans le tableau 6-A (évolution de l'activité fermentative test C1) et la figure 6-1 (stabilité dans des pâtons conservés à -20˚C).

Une très nette amélioration de la stabilité en congélation est observée pour les 3 mutants AT25, AT26 et AT28 (figure 6-1) ; ainsi, la souche témoin S47 conserve après 1 mois 54 % de sa force fermentative de référence, tandis que les mutants conservent, dans les mêmes conditions entre 80 et 87% de leurs forces fermentatives de référence. Par ailleurs, puisque les mutations n'ont pas entraîné de pénalisation importante de la force fermentative, les mutants obtiennent rapidement (dès 8 à 15 jours de conservation à -20˚C) des niveaux de force fermentative supérieurs au témoin S47 (tableau 6-A).

**Tableau 6-A :** Evolution de la force fermentative (test C1) en fonction de la durée de conservation à -20˚C.

| | Force fermentative après conservation à -20˚C | | | | | |
|---|---|---|---|---|---|---|
| | 1 jour | 9 jours | 15 jours | 23 jours | 30 jours | 45 jours |
| S47 | 115 | 102 | 89 | 77 | 62 | 47 |
| AT25 | 104 | 98 | 92 | 87 | 85 | 65 |
| AT26 | 108 | 105 | 99 | 94 | 90 | 69 |
| AT28 | 105 | 100 | 98 | 95 | 91 | 78 |

Ces résultats ont conduit à multiplier les essais pilotes sur ces 3 souches AT25, AT26, AT28 pour :

- sélectionner la meilleure souche, parmi ces 3 souches,
- vérifier que la souche sélectionnée n'avait aucun caractère gênant, notamment dans les différentes conditions de panification.

L'ensemble des résultats sur les 3 souches AT25, AT26 et AT28 ont montré que la meilleure souche donnant les résultats les plus réguliers suite à plus de 10 essais est la souche AT25.

Notamment cette souche AT25 dans le test R donne le même résultat que la souche S47.

Les levures de boulangerie obtenues avec cette souche AT25 ont été testées dans de nombreux procédés de panification, notamment en pâtes congelées, réalisés avec de nombreuses recettes différentes pour vérifier qu'elles n'apportaient aucun mauvais goût au pain, ce qui est le cas.

Les levures de boulangerie obtenues avec cette souche conduisent avec des pâtes congelées à une diminution spectaculaire du temps d'apprêt, c'est-à-dire du temps de fermentation après décongélation nécessaire pour obtenir un volume donné de pâte. Les caractéristiques essentielles de la souche AT25 sont les suivantes :

- rendement de production sur mélasse 50 inférieur de 2% par rapport à la souche S47
- obtention de levures pressées qui à 7,6% d'azote sur matières sèches donnent en moyenne 148 ml de $CO_2$ dans le test A1 en 2 heures, c'est-à-dire des levures un peu plus rapides (+ 10%) que la souche S47
- conservation 7 jours à 26˚C en sacs plastiques polyéthylène d'au moins 40% de l'activité initiale (après la production) dans le test A1
- aucune mauvaise odeur, aucun mauvais goût conféré aux pains, au contraire obtention dans des procédés comportant un long stockage des pâtes congelées (60 jours) de pains au goût franc sans goût de levure
- meilleure résistance au stress de la congélation conduisant dans le test C1 à une stabilité en conservation définie par le rapport :

$$\frac{\text{dégagement de CO}_2 \text{ selon test C1 après un mois (30 jours) de conservation à -20°C}}{\text{dégagement de CO}_2 \text{ selon test C1 après le premier jour de conservation à -20°C.}} \geq 80\%$$

**EXEMPLE 7 - Construction de nouvelles souches fil dérivées de S47 *fil400* = AT25**

L'analyse génétique des ségrégeants obtenus à partir de la souche AT25 laisse à penser que les mutations concernent plusieurs gènes.

La démarche suivante fut alors employée pour essayer d'obtenir des souches plus performantes à partir d'AT25.

Les ségrégeants de AT25 furent croisés selon leur signe avec l'haploïde de laboratoire W303-1A de signe a ou α. Les souches polyploïdes obtenues furent testées dans le test T7 après 2 semaines de congélation, et classées dans ce test par rapport à AT25 et S47 et au diploïde W303 obtenu par croisement des 2 haploïdes W303 de signes opposés.

On obtient dans cette première série de tests les valeurs suivantes pour les souches témoins :

|  | T7 | T6 |
|---|---|---|
|  | 2 semaines | consommation glucose après 90 minutes |
| AT25 | 28% | $\geq 5,0$ |
| S47 | 14% | $\geq 5,0$ |
| diploïde W303 | 12% | 3,5 |

On obtient ainsi 27 souches polyploïdes ayant un résultat égal ou supérieur à la souche S47 dans T7, dont 7 ont un résultat supérieur à la souche AT25. Toutes ces souches ont une consommation de glucose dans T6 supérieure au diploïde W303.

On croise alors entre eux les ségrégeants issus d'AT25 ayant donné par croisement avec un haploïde W303-1A, une souche polyploïde donnant dans le test T7 un résultat au moins égal à la souche S47.

On sélectionne les souches polyploïdes ainsi obtenues avec les tests T6 et T7, les critères étant les suivants :

- glucose consommé en 90 minutes au moins égal à 80% du glucose consommé par AT25 ou S47
- résultat dans T7 après 1 jour et après 3 semaines de congélation supérieur à celui d'AT25.

9 souches polyploïdes correspondant à cette définition ont été obtenues, dans un premier temps, 3 ont été déposées au C.N.C.M. Ces 3 souches ont donné les résultats suivants :

| Souche | T6 consommation de glucose en 90 minutes | T7 après 1 jour de congélation | T7 après 3 semaines de congélation | Réponse AMPc | T4 $\mu$max. sur mélasses 0,5% |
|---|---|---|---|---|---|
| AT251 | 5,8 | 68% | 38% | + | 0,6 |
| AT252 | 5,8 | 69% | 40% | - | 0,6 |
| AT254 | 5,7 | 66% | 40% | + | 0,6 |
| AT25 | 5,6 | 50% | 26% | ++ | 0,6 |
| S47 | 5,8 | 28% | 6% | ++ | 0,6 |

La souche AT251 a été déposée au C.N.C.M., 25 rue du Docteur Roux, F-75724, Paris cedex, sous le n° I-2222.

La souche AT252 a été déposée au C.N.C.M., 25 rue du Docteur Roux, F-75724, Paris cedex, sous le n° I-2223.

La souche AT254 a été déposée au C.N.C.M., 25 rue du Docteur Roux, F-75724, Paris cedex, sous le n° I-2224.

Ces résultats montrent que les 3 souches AT251, AT252 et AT254 sont de bonnes candidates pour donner sur pâtes congelées des résultats meilleurs que la souche AT25. Ces résultats montrent que le procédé consistant :

- à partir d'un mutant industriel ayant le phénotype fil, portant des mutations sur plusieurs gènes par rapport à la souche industrielle non mutée :
- à croiser les ségrégeants issus de ce mutant industriel avec un haploïde de souche de laboratoire pour sélectionner les ségrégeants du mutant industriel conférant aux polyploïdes obtenus avec la souche de laboratoire la ou les propriétés recherchées

- à croiser les ségrégeants du mutant industriel ainsi sélectionnés entre eux et à sélectionner les polyploïdes ainsi obtenus selon les critères du phénotype fil permet d'obtenir des souches industrielles fil améliorées.

Ce procédé est également un des objets de l'invention, ainsi que les souches du même type que les souches AT251, AT252, AT254 qu'il permet d'obtenir.

## Revendications

1. Procédé d'obtention de souches de levures *Saccharomyces cerevisiae* conservant une résistance au stress en présence de sucre fermentescible tel que le glucose, **caractérisé par le fait qu'**il comporte les étapes suivantes :

    on soumet les cellules d'une souche de départ à un traitement mutagène classique,
    on cultive les cellules ayant subi ledit traitement mutagène jusqu'à obtention d'une phase stationnaire,
    on incube lesdites cellules en phase stationnaire en présence d'au moins un sucre fermentescible choisi dans le groupe comprenant le glucose, le maltose et le saccharose, ce sucre étant présent en une quantité déterminée de manière à ce que les cellules passent en état de métabolisme actif (fermentation et/ou croissance) de ce sucre,
    on soumet lesdites cellules en état de métabolisme actif à un ou plusieurs stress conduisant à un taux de mortalité d'au moins 99% par rapport à la population de départ,
    on isole les cellules survivantes et
    on sélectionne celles de ces cellules survivantes qui répondent aux critères suivants qui caractérisent le phénotype fil, à savoir :

       - une croissance, évaluée par une production ou un rendement de production de biomasse sur sucre en un temps donné ou par un taux de croissance, en conditions identiques de culture, au moins égale à 80% de la souche de départ,
       - un dégagement de $CO_2$, ou une production de métabolite, en conditions identiques, au moins égal à 80% de la souche de départ,
       - une résistance au stress, correspondant à un taux de survie au moins 2 fois supérieur au taux de survie de la souche de départ, dans des conditions identiques de phase de croissance suivies d'un choc thermique de 20 minutes à 52˚C, ou au moins 1,5 fois supérieur au taux de survie de la souche de départ, dans des conditions identiques de phase de croissance suivie d'une congélation de 24 heures à -20˚C,
       - maintien de ces propriétés après des cultures répétées sur un milieu non sélectif, de manière à vérifier que le phénotype fil obtenu par la mutation est parfaitement stable et permanent.

2. Procédé selon la revendication 1 **caractérisé par le fait que** la souche de départ est une souche industrielle.

3. Procédé selon la revendication **2 caractérisé par le fait que** lorsqu'un mutant industriel fil comportant plusieurs mutations a été obtenu :

       - il est sporulé de manière à obtenir des ségrégeants ;
       - les ségrégeants issus du mutant industriel fil sont croisés avec une souche haploïde de laboratoire de manière à sélectionner les ségrégeants issus dudit mutant industriel fil donnant aux polyploïdes issus du croisement une propriété recherchée, comme la résistance à un stress donné ;
       - les ségrégeants ainsi sélectionnés sont croisés entre eux ;
       - les souches polyploïdes ainsi obtenues par croisement des ségrégeants industriels sélectionnés sont elles-mêmes sélectionnées sur la base des critères définis pour le phénotype fil dans la revendication 1, en recherchant les souches ayant au moins une propriété plus intéressante que la souche mutant fil de départ.

4. Procédé selon l'une des revendications 1 ou 2, **caractérisé par le fait que** les cellules en phase stationnaire, ayant subi le traitement mutagène, sont introduites dans des pâtons soumis à au moins 100 cycles de congélation/décongélation après une première fermentation du pâton de 30 minutes à 30˚C.

5. Procédé selon l'une des revendications 1 à 4, **caractérisé par le fait que** l'on sélectionne les souches qui ont de plus la propriété de conserver, en phase de croissance et/ou fermentation sur sucre fermentescible, au moins 50% de leur taux de survie en phase stationnaire mesuré dans de mêmes conditions après un choc à la chaleur ou à la congélation.

**6.** Souche industrielle de levure, du genre *Saccharomyces* ayant le phénotype fil tel que décrit à la revendication 1, susceptible d'être obtenue par le procédé selon l'une des revendications 1 à 5, **caractérisée par le fait qu'**elle donne un taux de survie, en phase de croissance sur sucre fermentescible, d'au moins 50%, après traitement thermique de 20 minutes à 52°C, la phase de croissance étant définie comme une remise en culture sur sucre fermentescible, tel que le glucose, 10 minutes à 30°C après la phase stationnaire.

**7.** Souche industrielle de levure selon la revendication 6**, caractérisée par le fait que** sa stabilité en congélation en pâtons incubés 30 minutes à 30°C avant congélation et contenant 20 g de farine, 15 g d'eau, 0,405 g de NaCl, 0,06 g de $(NH_4)_2SO_4$ et 160 mg de matière sèche de la souche considérée, cette stabilité étant mesurée par le ratio entre le dégagement $CO_2$ à 30°C après 1 mois de conservation à -20°C et le dégagement de $CO_2$ à 30°C après 1 jour de conservation à -20°C, est au moins égale à 80%.

**8.** Souche industrielle de levure selon l'une des revendications 6 à 7, **caractérisée par le fait que** sa perte de dégagement gazeux après séchage de la biomasse récoltée en phase proche de la phase exponentielle de croissance est au plus égale à 67%, de la perte de dégagement gazeux après séchage des levures obtenues avec la souche de départ correspondante ou à défaut d'une souche témoin ayant les mêmes caractéristiques.

**9.** Souche PVD1150 = M5 fil1 déposée au C.N.C.M. sous le n°I-2031 et sous le n°I-2203.

**10.** Souche KL1 = W303 fil2 déposée au C.N.C.M. sous le n°I-2032.

**11.** Souche FD51 = HL816 fil300 déposée au C.N.C.M. sous le n°I-2033.

**12.** Souche FDH16 - 22 = HL822 fil300 déposée au C.N.C.M. sous le n°I-2034.

**13.** Souche AT25 = S47 fi1400 déposée au C.N.C.M. sous le n°I-2035.

**14.** Souche AT28 = S47 fil500 déposée au C.N.C.M. sous le n°I-2036.

**15.** Souche AT251 déposée au C.N.C.M. sous le n° I-2222.

**16.** Souche AT252 déposée au C.N.C.M. sous le n° I-2223.

**17.** Souche AT254 déposée au C.N.C.M. sous le n° I-2224.

**18.** Gène *CDC35* = *CYR1* portant une mutation conférant le phénotype fil **caractérisé par le fait que** la mutation est un changement d'une base G en une base A dans la région du gène *CDC35/CYR1* codant pour le site catalytique de l'enzyme, équivalent à un changement d'un acide aminé acide en un acide aminé basique en position 1682 de la protéine.

**19.** Gène *GPR1* portant une mutation conférant le phénotype fil **caractérisé par le fait que** le Gène code pour version tronquée de la protéine Gpr1.

**20.** Gène GPR1 selon la revendication **19 caractérisé par le fait que** la mutation est un changement du codon qui code pour la tyrosine 316 de la protéine Gpr1 en codon stop.

**21.** Souche de levure transformée de manière à ce que certains des allèles du gène, objet d'une des revendications 18 à 20 portent une mutation conférant le phénotype fil à la souche.

**22.** Application d'une des souches objet de l'une des revendications 6 à 17 et 21 pour l'obtention de levures de panification destinées aux pâtes congelées.

**23.** Application d'une des souches objet de l'une des revendications 6 à 17 et **21** pour l'obtention de levures sèches de panification.

**24.** Application d'une des souches objet de l'une des revendications 6 à 17 et 21 pour l'obtention de levures de brasserie ou de vinification.

## EP 0 967 280 B1

**25.** Application d'une des souches objet de l'une des revendications 6 à 17 et 21 pour l'obtention de levures destinées à la production d'alcool.

**26.** Procédé selon la revendication 1, selon lequel on sélectionne les cellules survivantes qui présentent une croissance, évaluée par une production ou un rendement de production de biomasse sur sucre en un temps donné ou par un taux de croissance, en conditions identiques de culture, au moins égale à 90% de la souche de départ.

**27.** Procédé selon la revendication 1 ou 26, selon lequel on sélectionne les cellules survivantes qui présentent un dégagement de $CO_2$, ou une production de métabolite, en conditions identiques, au moins égal à 90% de la souche de départ.

**28.** Procédé selon l'une quelconque des revendications 1, 26 et 27, selon lequel on sélectionne les cellules survivantes présentant une résistance au stress, correspondant à un taux de survie au moins 5 fois supérieur au taux de survie de la souche de départ, dans des conditions identiques de phase de croissance suivies d'un choc thermique de 20 minutes à 52˚C, ou au moins 3 fois supérieur au taux de survie de la souche de départ, dans des conditions identiques de phase de croissance suivie d'une congélation de 24 heures à -20˚C.

**29.** Procédé selon la revendication 28, selon lequel on sélectionne les cellules survivantes présentant une résistance au stress, correspondant à un taux de survie au moins 10 fois supérieur au taux de survie de la souche de départ, dans des conditions identiques de phase de croissance suivies d'un choc thermique de 20 minutes à 52˚C, ou au moins 5 fois supérieur au taux de survie de la souche de départ, dans des conditions identiques de phase de croissance suivie d'une congélation de 24 heures à -20˚C.

**30.** Procédé selon la revendication 5, **caractérisé par le fait que** l'on sélectionne les souches qui ont de plus la propriété de conserver, en phase de croissance et/ou fermentation sur sucre fermentescible, au moins 70% de leur taux de survie en phase stationnaire mesuré dans de mêmes conditions après un choc à la chaleur ou à la congélation.

**31.** Procédé selon la revendication 30, **caractérisé par le fait que** l'on sélectionne les souches qui ont de plus la propriété de conserver, en phase de croissance et/ou fermentation sur sucre fermentescible, au moins 80% de leur taux de survie en phase stationnaire mesuré dans de mêmes conditions après un choc à la chaleur ou à la congélation.

**32.** Souche selon la revendication 6, **caractérisée par le fait qu'**elle donne un taux de survie, en phase de croissance sur sucre fermentescible d'au moins 70%.

**33.** Souche selon la revendication 32, **caractérisée par le fait qu'**elle donne un taux de survie, en phase de croissance sur sucre fermentescible d'au moins 75%.

**34.** Souche selon la revendication 7, **caractérisée par le fait que** sa stabilité est au moins égale à 90%.

**35.** Souche selon la revendication 8, **caractérisée par le fait que** sa perte de dégagement gazeux après séchage de la biomasse récoltée en phase proche de la phase exponentielle de croissance est au plus égale à 50% de la perte de dégagement gazeux après séchage des levures obtenues avec la souche de départ correspondante ou à défaut d'une souche témoin ayant les mêmes caractéristiques.

## Claims

**1.** Process for obtaining yeast strains of *Saccharomyces cerevisiae* conserving a stress resistance in the presence of fermentable sugar such as glucose, **characterized by** the fact that it includes the following steps:

- carrying out a classic mutagenic treatment on the cells of a starting strain,
- cultivating the cells which have undergone the said mutagenic treatment until a stationary phase is obtained,
- incubating said cells in stationary phase in the presence of at least one fermentable sugar selected from the group comprising glucose, maltose and sucrose, this sugar being present in a quantity such that the cells enter an active metabolic state (fermentation and/or growth) of this sugar,
- subjecting said cells in active metabolic state to one or several stresses leading to a mortality rate of at least 99% with respect to the starting population,
- isolating the surviving cells, and

- selecting those of the surviving cells which meet the following criteria which characterize the fil phenotype, namely:

> - a growth, evaluated by a production or a production yield of biomass over sugar in a given time or by a growth rate, under identical culture conditions, at least equal to 80% of the starting strain,
> - a $CO_2$ release, or a metabolite production, under identical conditions, at least equal to 80% of the starting strain,
> - a stress resistance, corresponding to a survival rate at least 2 times higher than the survival rate of the starting strain, under identical growth phase conditions followed by a heat shock of 20 minutes at 52˚C, or at least 1.5 times higher than the survival rate of the starting strain, under identical growth phase conditions followed by a freezing period of 24 hours at -20˚C,
> - maintenance of these properties after repeated cultures on a non-selective medium, in order to verify that the fil phenotype obtained by the mutation is perfectly stable and permanent.

2. The process according to claim 1, **characterized in that** the starting strain is an industrial strain.

3. The process according to claim 2, **characterized by** the fact that when an industrial fil mutant carrying several mutations is obtained:

> - it is sporulated to obtain segregants;
> - the segregants obtained from the industrial fil mutant are crossed with a laboratory haploid strain in order to select the segregants obtained from this industrial fil mutant giving to the polyploids obtained from the cross a sought property such as resistance to a given stress;
> - the segregants thus selected are crossed one with the other;
> - the polyploid strains thus obtained by crossing the selected industrial segregants are themselves selected on the basis of the criteria defined in claim 1 for the fil phenotype, by searching for strains having at least one property which is more interesting than the starting mutant fil strain.

4. The process according to claim 1 or 2, **characterized by** the fact that the cells in stationary phase obtained after the mutagenic treatment, are introduced into pieces of dough subjected to at least 100 cycles of freezing/thawing after a first fermentation of the pieces of dough of 30 minutes at 30˚C.

5. The process according to anyone of claims 1 to 4, **characterized by** the fact that the selected strains have the additional property of conserving, in growth and/or fermentation phase on fermentable sugar, at least 50% of their survival rate in stationary phase measured under the same conditions after a heat or freeze shock.

6. Industrial yeast strain of the genus *Saccharomyces* having the fil phenotype as defined in claim 1, obtainable by the process defined in anyone of claim 1 to 5, **characterized by** the fact that it has a survival rate, in growth phase on fermentable sugar, of at least 50% after heat treatment of 20 minutes at 52˚C, the growth phase being defined as a reculturing on fermentable sugar, such as glucose, 10 minutes at 30˚C after the stationary phase.

7. The industrial yeast strain according to claim 6, **characterized by** the fact that its stability to freezing in dough pieces incubated 30 minutes at 30˚C before freezing and containing 20 g of flour, 15 g water, 0.405 g NaCl, 0.06 g $(NH_4)_2SO_4$ and 160 mg of dry matter of the considered strain, this stability being measured by the ratio between the release of $CO_2$ at 30˚C after 1 month of conservation at -20˚C and the release of $CO_2$ at 30˚C after 1 day of conservation at -20˚C, is at least equal to 80%.

8. The industrial yeast strain according to anyone of claims 6 to 7, **characterized by** the fact that its loss of released gas after drying of the harvested biomass in a phase close to the exponential growth phase is at most equal to 67%, of the loss of released gas after drying yeasts obtained using the corresponding starting strain or a control strain having the same characteristics.

9. Strain PVD1150 = M5 fil1 deposited at the C.N.C.M. under n˚ I-2031 and under n˚ I-2203.

10. Strain KL1 = W303 fil2 deposited at the C.N.C.M. under n˚ I-2032.

11. Strain FD51 = HL816 fil300 deposited at the C.N.C.M. under n˚ I-2033.

12. Strain FDH16 = HL822 fil300 deposited at C.N.C.M. under n˚ I-2034.

13. Strain AT25 = S47 fil400 deposited at the C.N.C.M. under n˚ I-2035.

14. Strain AT28 = S47 fil500 deposited at the C.N.C.M. under n˚ I-2036.

15. Strain AT251 deposited at the C.N.C.M. under n˚ I-2222.

16. Strain AT252 deposited at the C.N.C.M. under n˚ I-2223.

17. Strain AT254 deposited at the C.N.C.M. under n˚ I-2224.

18. Gene *CDC35 = CYR1* carrying a mutation conferring the fil phenotype, **characterized by** the fact that the mutation is a change of a G base into an A base in the region of the gene *CDC35 = CYR1* coding for the catalytic site of the enzyme, equivalent to a change of an acidic amino acid into a basic amino acid at position 1682 of the protein.

19. Gene *GPR1* carrying a mutation conferring the fil phenotype, **characterized by** the fact that the gene codes for a truncated version of the *GRP1* protein.

20. The gene *GPR1* according to claim 19, **characterized by** the fact that the mutation is a change in the codon coding for the tyrosine 316 of the protein GRP1 into a stop codon.

21. Yeast strain transformed in a manner so that certain alleles of the gene, subject of claims 18 to 20, carry a mutation conferring the fil phenotype to the strain.

22. Use of one of the strains of one of the claims 6 to 17 and 21 for obtaining baker's yeasts for frozen pasta.

23. Use of one of the strains of one of the claims 6 to 17 and 21 for obtaining dry baker's yeasts.

24. Use of one of the strains of one of the claims 6 to 17 and 21 for obtaining brewery or winery yeasts.

25. Use of one of the strains of one of the claims 6 to 17 and 21 for obtaining yeasts for alcohol production.

26. The process according to claim 1, whereby the surviving cells presenting a growth, evaluated by a production or a production yield of biomass over sugar in a given time or by a growth rate, under identical culture conditions, of at least equal to 90% of the starting strain, are selected

27. The process according to claim 1 or 26, whereby the surviving cells presenting a $CO_2$ release, or a metabolite production, under identical conditions, at least equal to 90% of the starting strain, are selected.

28. The process according to anyone of claims 1, 26 and 27, whereby the surviving cells presenting a stress resistance, corresponding to a survival rate at least 5 times higher than the survival rate of the starting strain, under identical growth phase conditions followed by a heat shock of 20 minutes at 52˚C, or at least 3 times higher than the survival rate of the starting strain, under identical growth phase conditions followed by a freezing period of 24 hours at -20˚C, are selected.

29. The process according to claim 28, whereby the surviving cells presenting a stress resistance, corresponding to a survival rate at least 10 times higher than the survival rate of the starting strain, under identical growth phase conditions followed by a heat shock of 20 minutes at 52˚C, or at least 5 times higher than the survival rate of the starting strain, under identical growth phase conditions followed by a freezing period of 24 hours at -20˚C, are selected.

30. The process according to claim 5, **characterized by** the fact that the selected strains have the additional property of conserving, in growth and/or fermentation phase on fermentable sugar, at least 70% of their survival rate in stationary phase measured under the same conditions after a heat or freeze shock.

31. The process according to claim 30, **characterized by** the fact that the selected strains have the additional property of conserving, in growth and/or fermentation phase on fermentable sugar, at least 80% of their survival rate in stationary phase measured under the same conditions after a heat or freeze shock.

**32.** The industrial yeast strain according to claim 6, **characterized by** the fact that it has a survival rate, in growth phase on fermentable sugar, of at least 70%.

**33.** The industrial yeast strain according to claim 32, **characterized by** the fact that it has a survival rate, in growth phase on fermentable sugar, of at least 75%.

**34.** The industrial yeast strain according to claim 7, **characterized by** the fact that its stability is at least equal to 90%.

**35.** The industrial yeast strain according to claim 8, **characterized by** the fact that its loss of released gas after drying of the harvested biomass in a phase close to the exponential growth phase is at most equal to 50%, of the loss of released gas after drying yeasts obtained using the corresponding starting strain or a control strain having the same characteristics.

**Patentansprüche**

**1.** Verfahren um *Saccharomyces cerevisiae* Hefestämme zu erhalten, die in Gegenwart von fermentierbarem Zucker, wie der Glucose, Resistenz gegen Stress behalten, **dadurch gekennzeichnet, dass** es die folgenden Schritte umfasst:

die Zellen eines Ausgangsstamms einer klassischen mutagenen Behandlung unterziehen,
die Zellen, die der mutagenen Behandlung unterzogen worden sind, bis zum Erreichen einer stationären Phase kultivieren,
die Zellen in der stationären Phase in Gegenwart von mindestens einem fermentierbaren Zucker, ausgewählt aus der Gruppe, umfassend die Glucose, die Maltose und die Saccharose, inkubieren, wobei der Zucker in einer Menge vorhanden ist, die so bestimmt ist, dass die Zellen auf den Zustand des aktiven Metabolismus (Fermentation und/oder Wachstum) des Zuckers umstellen,
die Zellen im Zustand des aktiven Metabolismus einem oder mehreren Stress(en) unterwerfen, was zu einer Mortalitätsrate von mindestens 99%, bezogen auf die Ausgangspopulation, führt,
die überlebenden Zellen isolieren und
die Zellen der überlebenden Zellen selektieren, die die folgenden Kriterien, die den fil-Phänotyp kennzeichnen, erfüllen, und zwar:

- ein Wachstum von mindestens 80% des Ausgangsstamms, unter identischen Kulturbedingungen, gemessen durch eine Produktion oder einen Produktionsertrag an Biomasse aus Zucker in einer bestimmten Zeit oder durch eine Wachstumsrate,
- eine $CO_2$-Abgabe oder eine Metabolitproduktion von mindestens 80% des Ausgangsstamms unter identischen Bedingungen,
- eine Resistenz gegen Stress, die einer Überlebensrate entspricht, die mindestens 2 mal so hoch ist wie die Überlebensrate des Ausgangsstamms, unter identischen Bedingungen der Wachstumsphase gefolgt von einem thermischen Schock von 20 Minuten bei 52˚C, oder die mindestens 1,5 mal so hoch ist, wie die Überlebensrate des Ausgangsstamms, unter identischen Bedingungen der Wachstumsphase gefolgt von Einfrieren für 24 Stunden bei -20˚C,
- Aufrechterhaltung dieser Eigenschaften nach wiederholten Kultivierungen in einer nicht selektiven Umgebung, um zu überprüfen, dass der durch die Mutation erhaltene fil-Phänotyp vollkommen stabil und beständig ist.

**2.** Verfahren nach Anspruch 1, **dadurch gekennzeichnet, dass** der Ausgangsstamm ein Industriestamm ist.

**3.** Verfahren nach Anspruch 2, **dadurch gekennzeichnet, dass,** wenn eine industrielle fil-Mutante erhalten worden ist, die mehrere Mutationen aufweist:

- sie sporuliert wird, so dass Segreganten erhalten werden;
- die aus der industriellen fil-Mutante stammenden Segreganten mit einem haploiden Laborstamm gekreuzt werden, so dass die Segreganten selektiert werden, die aus der industriellen fil-Mutante stammen, was den aus der Kreuzung stammenden Polyploiden eine gesuchte Eigenschaft verleiht, wie die Resistenz gegen einen bestimmten Stress;
- die so selektierten Segreganten untereinander gekreuzt werden;

EP 0 967 280 B1

- die so durch Kreuzung selektierter industrieller Segreganten erhaltenen polyploiden Stämme selbst auf der Basis der für den fil-Phänotyp in Anspruch 1 definierten Kriterien selektiert werden, wobei Stämme gesucht werden, die mindestens eine interessantere Eigenschaft haben als der Ausgangsstamm der fil-Mutante.

4. Verfahren nach einem der Ansprüche 1 oder 2, **dadurch gekennzeichnet, dass** die Zellen, die der mutagenen Behandlung unterzogen worden sind, in der stationären Phase in Teigportionen eingefügt werden, die nach einer ersten Fermentierung der Teigportionen von 30 Minuten bei 30˚C mindestens 100 Einfrier/Auftauzyklen unterworfen werden.

5. Verfahren nach einem der Ansprüche 1 bis 4, **dadurch gekennzeichnet, dass** Stämme selektiert werden, die außerdem die Eigenschaft haben, in der Wachstums- und/oder Fermentationsphase mit fermentierbarem Zucker mindestens 50% ihrer Überlebensrate in der stationären Phase beizubehalten, gemessen unter den gleichen Bedingungen nach einem Hitze- oder Einfrierschock.

6. Industriestamm von Hefe der Gattung *Saccharomyces,* der den fil-Phänotyp, wie in Anspruch 1 beschrieben, aufweist, erhältlich durch das Verfahren nach einem der Ansprüche 1 bis 5, **dadurch gekennzeichnet, dass** er nach Wärmebehandlung für 20 Minuten bei 52˚C eine Überlebensrate in der Wachstumsphase mit fermentierbarem Zucker von mindestens 50% aufweist, wobei die Wachstumsphase als Wiederaufnahme der Kultivierung mit fermentierbarem Zucker, wie der Glucose, 10 Minuten bei 30˚C nach der stationären Phase definiert ist.

7. Industriestamm von Hefe nach Anspruch 6, **dadurch gekennzeichnet, dass** seine Stabilität beim Einfrieren in Teigportionen, die vor dem Einfrieren 30 Minuten bei 30˚C inkubiert werden und die 20 g Mehl, 15 g Wasser, 0,405 g NaCl, 0,06 g $(NH_4)_2SO_4$ und 160 mg Trockenmaterial des betreffenden Stamms enthalten, mindestens 80% ist, wobei die Stabilität, als Verhältnis zwischen der $CO_2$-Abgabe bei 30˚C nach 1 Monat Aufbewahrung bei -20˚C und der $CO_2$-Abgabe bei 30˚C nach 1 Tag Aufbewahrung bei -20˚C, gemessen wird.

8. Industriestamm von Hefe nach einem der Ansprüche 6 bis 7, **dadurch gekennzeichnet, dass** sein Verlust an Gasabgabe nach dem Trocknen der Biomasse, die in einer Phase, die nahe an der exponentiellen Wachstumsphase ist, geerntet wurde, höchstens 67% des Verlusts an Gasabgabe nach dem Trocknen der Hefen ist, die mit dem entsprechenden Ausgangsstamm erhalten wurden, oder anderenfalls eines Vergleichsstamms, der die gleichen Merkmale aufweist.

9. Stamm PVD1150 = M5 fil1 hinterlegt bei der C.N.C.M. unter der Nr. 1-2031 und unter der Nr. I-2203.

10. Stamm KL1 = W303 fil2 hinterlegt bei der C.N.C.M. unter der Nr. 1-2032.

11. Stamm FD51 = HL816 fil300 hinterlegt bei der C.N.C.M. unter der Nr. I-2033.

12. Stamm FDH16-22 = HL822 fil300 hinterlegt bei der C.N.C.M. unter der Nr. I-2034.

13. Stamm AT25 = S47 fil400 hinterlegt bei der C.N.C.M. unter der Nr. 1-2035.

14. Stamm AT28 = S47 fil500 hinterlegt bei der C.N.C.M. unter der Nr. 1-2036.

15. Stamm AT251 hinterlegt bei der C.N.C.M. unter der Nr. I-2222.

16. Stamm AT252 hinterlegt bei der C.N.C.M. unter der Nr. 1-2223.

17. Stamm AT254 hinterlegt bei der C.N.C.M. unter der Nr. I-2224.

18. Gen CDC35 = CYR1, das eine Mutation trägt, die den fil-Phänotyp verleiht, **dadurch gekennzeichnet, dass** die Mutation eine Änderung einer Base G zu einer Base A in der Region des Gens CDC35/CYR1, das für die katalytische Stelle des Enzyms kodiert, ist, was einer Änderung einer sauren Aminosäure zu einer basischen Aminosäure an der Position 1682 des Proteins äquivalent ist.

19. Gen GPR1, das eine Mutation trägt, die den fil-Phänotyp verleiht, **dadurch gekennzeichnet, dass** das Gen für eine verkürzte Version des Proteins Gpr1 kodiert.

36

**20.** Gen GPR1 nach Anspruch 19, **dadurch gekennzeichnet, dass** die Mutation eine Änderung des Codons, das für das Tyrosin 316 des Proteins Gpr1 kodiert, zu einem Stop-Codon ist.

**21.** Hefestamm, der transformiert ist, so dass bestimmte Allele des Gens, das Gegenstand eines der Ansprüche 18 bis 20 ist, eine Mutation tragen, die dem Stamm den fil-Phänotyp verleiht.

**22.** Verwendung eines der Stämme, die Gegenstand eines der Ansprüche 6 bis 17 und 21 sind, um Brothefen zu erhalten, die für eingefrorenen Teig bestimmt sind.

**23.** Verwendung eines der Stämme, die Gegenstand eines der Ansprüche 6 bis 17 und 21 sind, um trockene Brothefen zu erhalten.

**24.** Verwendung eines der Stämme, die Gegenstand eines der Ansprüche 6 bis 17 und 21 sind, um Brau- oder Weinhefen zu erhalten.

**25.** Verwendung eines der Stämme, die Gegenstand eines der Ansprüche 6 bis 17 und 21 sind, um Hefen zu erhalten, die für die Alkoholproduktion bestimmt sind.

**26.** Verfahren nach Anspruch 1, wonach die überlebenden Zellen, die ein Wachstum von mindestens 90% des Ausgangsstamms unter identischen Kulturbedingungen aufweisen, gemessen durch eine Produktion oder einen Produktionsertrag an Biomasse aus Zucker in einer bestimmten Zeit oder durch eine Wachstumsrate, selektiert werden.

**27.** Verfahren nach Anspruch 1 oder 26, wonach die überlebenden Zellen, die eine $CO_2$-Abgabe oder eine Metabolitproduktion von mindestens 90% des Ausgangsstamms unter identischen Bedingungen aufweise, selektiert werden.

**28.** Verfahren nach einem der Ansprüche 1, 26 und 27, wonach die überlebenden Zellen, die eine Resistenz gegen Stress aufweisen, die einer Überlebensrate entspricht, die mindestens 5 mal so hoch ist, wie die Überlebensrate des Ausgangsstamms unter identischen Bedingungen der Wachstumsphase, gefolgt von einem thermischen Schock von 20 Minuten bei 52˚C, oder die mindestens 3 mal so hoch ist, wie die Überlebensrate des Ausgangsstamms unter identischen Bedingungen der Wachstumsphase, gefolgt von Einfrieren für 24 Stunden bei -20˚C, selektiert werden.

**29.** Verfahren nach Anspruch 28, wonach die überlebenden Zellen, die eine Resistenz gegen Stress aufweise, die einer Überlebensrate entspricht, die mindestens 10 mal so hoch ist, wie die Überlebensrate des Ausgangsstamms unter identischen Bedingungen der Wachstumsphase, gefolgt von einem thermischen Schock von 20 Minuten bei 52˚C, oder die mindestens 5 mal so hoch ist, wie die Überlebensrate des Ausgangsstamms unter identischen Bedingungen der Wachstumsphase, gefolgt von Einfrieren für 24 Stunden bei -20˚C, selektiert werden.

**30.** Verfahren nach Anspruch 5, **dadurch gekennzeichnet, dass** die Stämme selektiert werden, die außerdem die Eigenschaft haben, in der Wachstums- und/oder Fermentationsphase mit fermentierbarem Zucker mindestens 70% ihrer Überlebensrate in der stationären Phase beizubehalten, gemessen unter den gleichen Bedingungen nach einem Hitze oder Einfrierschock.

**31.** Verfahren nach Anspruch 30, **dadurch gekennzeichnet, dass** die Stämme selektiert werden, die außerdem die Eigenschaft haben, in der Wachstums- und/oder Fermentationsphase mit fermentierbarem Zucker mindestens 80% ihrer Überlebensrate in der stationären Phase beizubehalten, gemessen unter den gleichen Bedingungen nach einem Hitze oder Einfrierschock.

**32.** Stamm nach Anspruch 6, **dadurch gekennzeichnet, dass** er eine Überlebensrate in der Wachstumsphase mit fermentierbarem Zucker von mindestens 70% aufweist.

**33.** Stamm nach Anspruch 32, **dadurch gekennzeichnet, dass** er eine Überlebensrate in der Wachstumsphase mit fermentierbarem Zucker von mindestens 75% aufweist.

**34.** Stamm nach Anspruch 7, **dadurch gekennzeichnet, dass** seine Stabilität mindestens 90% ist.

**35.** Stamm nach Anspruch 8, **dadurch gekennzeichnet, dass** sein Verlust an Gasabgabe nach dem Trocknen der Biomasse, die in einer Phase, die nahe an der exponentiellen Wachstumsphase ist, geerntet wurde, höchstens 50% des Verlusts an Gasabgabe nach dem Trocknen der Hefen ist, die mit dem entsprechenden Ausgangsstamm erhalten wurden, oder anderenfalls eines Vergleichsstamms, der die gleichen Merkmale aufweist.

**Figure 1-1** : Croissance des souches M5 et M5 *fil1* sur milieu YPD à 30°C et sous agitation (180 tours/min)

**Figure 1-2** : Réponse AMPc (Unités Arbitraires) des souches PVD1150 et M5 après ajout de glucose (à 100 mM) à des cellules cultivées sur milieu glycérol jusqu'à l'obtention de la phase stationnaire.

**Figure 1-3** : Suivi de la dégradation du tréhalose (Unités Arbitraires) dans les souches PVD1050, PVD1150 et leurs témoins respectifs M5 *hxk2Δ* et M5, après une induction par le glucose (200 mM) sur des cellules en phase stationnaire.

**Figure 1-4** : Taux de survie des souches HL8.16 leu2 et HL816 fil300 après un choc thermique de 30 minutes à 52°C. Les cellules sont cultivées jusqu'à l'obtention de la phase stationnaire, puis incubées à 30°C en présence de glucose pendant 0 à 90 minutes, avant d'être traitées thermiquement.

**Figure 1-5** : Taux de survie des souches HL8.16 leu2 et HL816 fil300 après une congélation de 12 jours à -25°C.

**Figure 1-6** : Taux de tréhalose (Unités Arbitraires) dans les souches HL8.16 leu2 et HL816 fil300. Les souches sont cultivées jusqu'à l'obtention de la phase stationnaire, puis du glucose (100 mM) est ajouté à t=0.

**Figure 1-7** : Réponse AMPc (Unités Arbitraires) après induction par le glucose (100 mM) dans les souches HL8.16 leu2 et HL816 fil300 cultivées jusqu'à l'obtention de la phase stationnaire. L'ajout initial de 3 mM de glucose permet de s'affranchir de la réponse AMPc liée à l'acidification intracellulaire.

**Figure 2-1** : Suivi de la dégradation de tréhalose (Unités Arbitraires) après une induction par le glucose à t = 0 (sur des cellules en phase stationnaire).

**Figure 2-2** : Suivi de la réponse AMPc (Unités Arbitraires) après une induction (à t = 0) par le glucose sur des cellules en phase exponentielle de croissance sur maltose. a) témoin S47 et mutant AT25 ; b) témoin S47 et mutants AT26, AT28 et AT31.

**Figure 3-1** : Stratégie de gap-filling utilisée pour l'isolement du gène portant la mutation *fil1* dans la souche PVD1150.

**Figure 3-2** : Carte physique du vecteur pUC18-CYR1$^{mut}$ -URA3 [Sn].

**Figure 6-1** : Stabilité en congélation mesurée par le ratio entre la force au jour de mesure et la force après 1 jour de conservation à -20°C.

**EP 0 967 280 B1**

## RÉFÉRENCES CITÉES DANS LA DESCRIPTION

### Documents brevets cités dans la description

- EP 0451896 A
- EP 0838520 A, Hino
- WO 9701626 A
- EP 196233 A
- US 4547374 A
- EP 0388262 A
- JP 08051917 B
- US 4318929 A
- US 4318930 A
- US 4396632 A

### Littérature non-brevet citée dans la description

- **Attfield.** la résistance au stress des levures du genre Saccharomyces. *Nature Biotechnology,* Décembre 1997, vol. 15, 1351-1357
- **Attfield.** *Nature Biotechnology,* 1997, vol. 15, 1351-1357
- **Winde et al.** Yeast Stress Responses. Springer, 1997, 7-52
- **Werner-Washburne et al.** *Microbiol.Rev.,* 1993, vol. 57, 383-401
- **Iida.** *Mol. Cell. Biol.,* 1988, vol. 8, 5555-5560
- **Matsumoto et al.** *Yeast,* 1985, vol. 1, 15-24
- **Shin et al.** *Mol. Cell. Biol.,* 1987, vol. 7, 244-250
- **Park et al.** *Appl. Envir. Microbiol.,* 1997, vol. 63, 3818-3824
- **Hitara et al.** *Mol. Gen. Genet.,* 1995, vol. 249, 257-264
- **Broach ; Deschenes.** *Adv. Cancer Res.,* 1990, vol. 54, 79-139
- **Thevelein.** *Mol. Microbiol.,* 1991, vol. 5, 1301-1307
- **Thevelein.** *Antonie Leeuwenhoek, J. Microbiology,* 1992, vol. 62, 109-130
- **Kataoka et al.** *Cell,* 1985, vol. 43, 493-505
- **Nikawa et al.** *Mol. Cell. Biol.,* 1997, vol. 7, 3629-3636
- **Sass et al.** *Proc. Natl. Acad. Sci. USA,* 1986, vol. 83, 9303-9307
- **Toda et al.** *Cell,* 1985, vol. 40, 27-36
- **Boy-Marcotte et al.** *Mol. Biol. Cell,* 1996, vol. 7, 529-539
- **Camonis et al.** *EMBO J.,* 1986, vol. 5, 375-380
- **Toda et al.** *Mol. Cell. Biol.,* 1987, vol. 7, 1371-1327
- **Toda et al.** *Cell,* 1987, vol. 50, 277-287
- **Cameron et al.** *Cell,* 1988, vol. 53, 555-566
- **Hottiger et al.** *FEBS Lett.,* 1989, vol. 255, 431-434
- **Ma et al.** *Microbiol.,* 1997, vol. 143, 3451-3459
- **Iida.** *Mol. Cell. Biol.,* 1988, vol. 8, 5559
- **Tatchell et al.** *Proc. Natl. Acad. Sci.,* 1985, vol. 82, 3785-3789
- **J.F. Canon et al.** *Genetics,* Juin 1986, vol. 113, 247-264
- **De Virgilio et al.** *Eur. J. Biochem.,* 1994, vol. 219, 179-186
- **Iwakashi et al.** *Lett. Appl. Microbiol.,* 1997, vol. 25, 43-47
- **Wiemken.** *Antonie Leeuwenhoek, J. Microbiology,* 1990, vol. 58, 209-217
- **Van Laere.** *FEMS Microbiol. Rev.,* 1989, vol. 63, 201-210
- **Wiemkem.** *Antonie Leeuwenhoek, J. Microbiology,* 1990, vol. 58, 209-217
- **Crowe et al.** *Anhydrobiosos Annu. Rev. Physiol.,* 1992, vol. 54, 579-599
- **van der Plaat.** *Biochem. Biophys. Res. Commun.,* 1974, vol. 56, 580-587
- **Kopp et al.** *J. Biol. Chem.,* 1993, vol. 268, 4766-4774
- **Van Dijck et al.** *Appl. Environ. Microbiol.,* 1995, vol. 61, 109-115
- **Crauwels et al.** *Microbiol.,* 1997, vol. 143, 2627-2637
- **de Winde et al.** Yeast Stress Responses. Springer, 1997, 7-52
- **Praekelt ; Maecock.** *Mol. Gen. Genet.,* 1990, vol. 223, 97-106
- **Werner-Washburne et al.** *J. Bacteriol.,* 1989, vol. 171, 2680-2688
- **McKown et al.** *Cryobiology,* 1991, vol. 28, 474-482
- *Nature Biotechnology,* 1997
- *Nature,* 1992, vol. 357, 38-44
- **Burrows ; Harrison.** *Journal of the Institute of Brewing,* Janvier 1959, vol. LXV, 1

- **J.F. Cannon et al.** *Genetics,* Juin 1986, vol. 113, 250
- **Iida.** *Mol.Cell.Biol.,* Décembre 1988, 5555-5560
- **Spencer et al.** Yeast a practical approach
- **Schaaff et al.** *Curr. Genet.,* 1989, vol. 15, 75-81
- **Thomas ; Rothstein.** *Cell,* 1989, vol. 56, 619-630
- **Xue et al.** *EMBO J.,* 1998, vol. 17 (7), 1996-2007
- **Schaaff.** *Curr. Genet.,* 1989, vol. 15, 75-81
- **Sherman.** Methods in yeast Genetics. Cold Spring Harbor Laboratory Press, 1986
- **Thévelein.** *J. Gen. Microbiol.,* 1987, vol. 133, 2197-2205
- **Neves.** *FEBS Lett.,* 1991, vol. 283, 19-22
- **Van Dijck.** *Appl. Environ. Microbiol,* 1995, vol. 61, 109-115
- **Sherman et al.** Methods in yeast Genetics. Cold Spring Harbor Laboratory Press, 1986
- Yeast genetics. **Spencer J.F.T. ; Spencer D.M.** Yeast a practical approach. 1988
- **J. Sambrook ; E.F. Fritsch ; T. Maniatis.** MOLECULAR CLONING. Cold Spring Harbor Laboratory Press, 1989
- **Gietz R.D. ; Sugino A.** *Gene,* 1988, vol. 74, 527-534
- **Iwasaki, T.** *Gene,* 1991, vol. 109, 81-87
- **Orr-Weaver, T.L.** *Methods Enzymol.,* 1983, vol. 101, 228-245
- **Jones ; Prakash.** *Yeast,* 1990, 363-366
- **Schaaf.** *Curr. Genet.,* 1989, vol. 15, 78-81
- **Toda.** *Cell,* 1985, vol. 40, 27-36
- **Xue, Y.** *EMBO Journal,* 1998, vol. 17, 1996-2007